# EUROPEAN PATENT SPECIFICATION

(11) **EP 2 002 021 B1**
(45) Date of publication and mention of the grant of the patent: **12.10.2011**
(21) Application number: 07758759.0
(22) Date of filing: 16.03.2007
(51) Int. Cl.: G01N 33/50, G01N 33/542, C12Q 1/68, C12N 15/10, C12Q 1/37

(54) **METHODS FOR ASSAYING PROTEIN-PROTEIN INTERACTION**
VERFAHREN ZUR UNTERSUCHUNG VON PROTEIN-PROTEIN-INTERAKTIONEN
PROCÉDÉS DE TEST D'INTERACTIONS PROTÉINE/PROTÉINE

(30) Priority: 16.03.2006 US 782980 P
(43) Date of publication of application: 17.12.2008
(73) Proprietor: Life Technologies Corporation, Carlsbad, CA 92008 (US)
(72) Inventor: LEE, Kevin, J., New York, NY 10025 (US); POLLOK, Brian, A., Madison, WI 53593 (US); ZHONG, Zhong, Middleton, WI 53562 (US)
(74) Representative: Harrison Goddard Foote
(86) International application number: PCT/US2007/064243
(87) International publication number: WO 2007/127538

(56) References cited:
- WO-A-2005/007822
- WO-A2-03/076932
- US-A1- 2002 028 433
- US-A1- 2002 106 698

## Description

### FIELD OF THE INVENTION

This invention relates to methods for determining if one or more test compounds modulate specific protein/protein interactions. In specific embodiments, the interactions are determined as a result of intracellular or extracellular activities of molecules, such as proteins or portions of proteins. Those intracellular activities are set into motion by the aforesaid interaction, *e.g.*, between molecules of interest. The invention also relates, in part, to determining if a particular substance referred to as the test compound modulates the interaction of two or more specific proteins of interest, *e.g.*, via determining activation of a reporter gene in a cell, where the activation, or lack thereof, results from the modulation or its absence. In particular embodiments, determination occurs using transformed or transfected cells, which are also a feature of the invention, as are the agents used to transform or transfect them

### BACKGROUND AND RELATED ART

The study of protein/protein interaction, as exemplified, *e.g.*, by the identification of ligands for receptors, is an area of great interest. Even when a ligand or ligands for a given receptor are known, there is interest in identifying more effective or more selective ligands. GPCRs will be discussed herein as a non-exclusive example of a class of proteins which can be studied in this way. Other classes of proteins that can be studied in these ways include, but are not limited to, cellular receptors, ion channels, growth factor receptors, and cytokine receptors.

The G-protein coupled receptors, or "GPCRs" hereafter, are the largest class of cell surface receptors known for humans. Among the ligands recognized by GPCRs are hormones, neurotransmitters, peptides, glycoproteins, lipids, nucleotides, and ions. They also act as receptors for light, odors, pheromones, and taste. Given these various roles, it is perhaps not surprising that they are the subject of intense research, seeking to identify drugs useful in various conditions. The success rate has been phenomenal. Indeed, Howard, *et al., Trends Pharmacol. Sci., 22*:132-140 (2001) estimate that over 50% of marketed drugs act on such receptors. "GPCRs" as used herein, refers to any member of the GPCR superfamily of receptors characterized by a seven-transmembrane domain (7TM) structure. Examples of these receptors include, but are not limited to, the class A or "rhodopsin-like" receptors; the class B or "secretin-like" receptors; the class C or "metabotropic glutamate-like" receptors; the Frizzled and Smoothened-related receptors; the adhesion receptor family or EGF-7TM / LNB-7TM receptors; adiponectin receptors and related receptors; and chemosensory receptors including odorant, taste, vomeronasal and pheromone receptors. As examples, the GPCR superfamily in humans includes but is not limited to those receptor molecules described by Vassilatis, et al., Proc. Natl. Acad. Sci. USA, 100:4903-4908 (2003); Takeda, et al., FEBS Letters, 520:97-101 (2002); Fredricksson, et al., Mol. Pharmacol., 63:1256-1272 (2003); Glusman, et at., Genome Res., 11:685-702 (2001); and Zozulya, et al., Genome Biol., 2:0018.1-0018.12 (2001).

The mechanisms of action by which GPCRs function has been explicated to some degree. In brief, when a GPCR binds a ligand, a conformational change results, stimulating a cascade of reactions leading to a change in cell physiology. It is thought that GPCRs transduce signals by modulating the activity of intracellular, heterotrimeric guanine nucleotide binding proteins, or "G proteins". The complex of ligand and receptor stimulates guanine nucleotide exchange and dissociation of the G protein heterotrimer into a and βγ subunits.

Both the GTP-bound a subunit and the βγ dimer can act to regulate various cellular effector proteins, including adenylyl cyclase and phospholipase C (PLC). In conventional cell based assays for GPCRs, receptor activity is monitored by measuring the output of a G-protein regulated effector pathway, such as the accumulation of cAMP that is produced by adenylyl cyclase, or the release of intracellular calcium, which is stimulated by PLC activity.

In some cases, conventional G-protein based, signal transduction assays have been difficult to develop for some targets, as a result of two major issues.

First, different GPCRs are coupled to different G protein regulated signal transduction pathways, and G-protein based assays are dependent on knowing the G-protein specificity of the target receptor, or require engineering of the cellular system, to force coupling of the target receptor to a particular effect or pathway. Second, all cells express a large number of endogenous GPCRs, as well as other signaling factors. As a result, the effector pathways that are measured may be modulated by other endogenous molecules in addition to the target GPCR, potentially leading to false results.

Regulation of G-protein activity is not the only result of ligand/GPCR binding. Luttrell, et al., J. Cell Sci., 115:455-465 (2002), and Ferguson, Pharmacol. Rev., 53:1-24 (2001), both of which are incorporated by reference, review other activities which lead to termination of the GPCR signal. These termination processes prevent excessive cell stimulation, and enforce temporal linkage between extracellular signal and corresponding intracellular pathway.

In the case of binding of an agonist to GPCR, serine and threonine residues at the C terminus of the GPCR molecule are phosphorylated. This phosphorylation is caused by the GPCR kinase, or "GRK," family. Agonist complexed, C-terminal phosphorylated GPCRs interact with arrestin family members, which "arrest" receptor signaling. This binding inhibits coupling of the receptor to G proteins, thereby targeting the receptor for internalization, followed by degradation and/or recycling. Hence, the binding of a ligand to a GPCR can be said to "modulate" the interaction between the GPCR and arrestin protein, since the binding of ligand to GPCR causes the arrestin to bind to the GPCR, thereby modulating its activity. Hereafter, when "modulates" or any form thereof is used, it refers simply to some change in the way the two proteins of the invention interact, when the test compound is present, as compared to how these two proteins interact, in its absence. For example, the presence of the test compound may strengthen or enhance the interaction of the two proteins, weaken it, inhibit it, or lessen it in some way, manner or form which can then be detected.

This background information has led to alternate methods for assaying activation and inhibition of GPCRs. These methods involve monitoring interaction with arrestins. A major advantage of this approach is that no knowledge of G-protein pathways is necessary.

Oakley, et al., Assay Drug Dev. Techno/., 1:21-30 (2002) and U.S. Patent Nos. 5,891,646 and 6,110,693, describe assays where the redistribution of fluorescently labelled arrestin molecules in the cytoplasm to activated receptors on the cell surface is measured. These methods rely on high resolution imaging of cells, in order to measure arrestin relocalization and receptor activation. It will be recognized by the skilled artisan that this is a complex, involved procedure.

Various other U.S. patents and patent applications dealing with these points have issued and been filed. For example, U.S. Patent No. 6,528,271 to Bohn, et al.*,* deals with assays for screening for pain controlling medications, where the inhibitor of β-arrestin binding is measured. Published U.S. patent applications, such as 2004/0002119, 2003/0157553, 2003/0143626, and 2002/0132327, and U.S. Patent No. 7,049,076 describe different forms of assays involving GPCRs. Published application 2002/0106379 describes a construct which is used in an example which follows; however, it does not teach or suggest the invention described herein.

Protein complementation methods are becoming a common method for studying the dynamics of protein-protein interactions in cells. (Remy and Michnick, Nature Methods 3(12):977-979 (2006)). In this strategy, two proteins of interest are fused to complementary fragments of a reporter protein. If the proteins interact, the reporter fragments are brought together, fold into the native structure and the PCA reporter activity is reconstituted (*e.g.*, see Michnick, Curr. Opin. Struct Biol. 11:472-477 (2001). Some related methods are based on fluorescent proteins because a signal is provided by the intrinsic fluorophore (*e.g.* see Ghosh et al., Am. Chem. Soc. 122:5658-5659 (2000); Hu et al., Mo/. Cell 9:789-798 (2002); Remy & Michnick, Methods 32:381-388 (2004); Remy et al., Nat Cell Biol. 6:358-365 (2004); Magliery et al., J. Am. Chem. Soc. 127:146-157 (2005); and Macdonald et al., Nat Chem. Biol. 2:329-337 (2006)).
WO 2005/007822 discloses a method for determining if a test compound, or a mix of compounds, modulates the interaction between two proteins of interest. The determination is made possible via the use of two recombinant molecules, one of which contains the first protein a cleavage site for a proteolytic molecules, and an activator of a gene. The second recombinant molecule includes the second protein and the proteolytic molecule. If the test compound binds to the first protein, a reaction is initiated whereby the activator is cleaved, and activates a reporter gene.
WO 03/076932 describes a method for detecting and analyzing protein interactions in a cell, comprising the following steps: a) providing the activity of at least one enzyme selected from the group consisting of recombinases and proteases in the cell following a protein interaction; b) continued generation of an active reporter protein in the respective cell following the enzyme activity from step a) for a period of time exceeding that of the protein interaction from step a), and; c) producing a detection signal using the reporter proteins generated in b). It also describes reverse configurations of the above method for detecting and analyzing protein interactions in a cell, whereby following the induced dissociation of a defined interaction between proteins, the activity of at least one enzyme selected from the group consisting of recombinases and proteases is provided in the cell and is converted into a permanent detection signal of the cell.

It is an object of the invention to develop assays (e.g. a simpler assay) for monitoring and/or determining modulation of specific protein/protein interactions, where the proteins include but are not limited to, membrane bound proteins, such as receptors, GPCRs, ion channels, growth factor receptors, and cytokine receptors. How this is accomplished will be seen in the descriptions and examples which follow.

### SUMMARY OF THE INVENTION

The present invention relates, in part, to protein to protein interactions of at least two proteins. In some embodiments of the invention, an interaction of the at least two proteins results in a detectable signal, *e.g.*, fluorescent, colorimetric, etc. In some embodiments of the invention, the interaction of the at least two proteins results in bringing two other proteins (*e.g.*, a protease and its recognition site) within close molecular proximity. The interaction of the two other proteins may be detected. For example, this feature can then be exploited to cleave and release a detectable protein(s) or protein fragments associated with one or more test proteins.

The invention relates, in part, to methods for determining if a test compound, or a mix of compounds, modulates an interaction between at least two proteins. In some embodiments, this determination is made possible via the use of two recombinant molecules, e.g, one of which contains a first protein cleavage site for a proteolytic molecules, and an activator of a gene. A second recombinant molecule may include a second protein and the proteolytic molecule. Various other formats are provided by the invention.

Thus, in accordance with the present invention, there is provided, in part, methods for determining if a test compound modulates a specific protein/protein interaction of interest. Some methods of the invention comprise contacting a compound to a cell which has been transformed or transfected with (a) a nucleic acid molecule which encodes a first fusion protein, the nucleic acid molecule comprising, (i) a nucleotide sequence which encodes a first test protein, (ii) a nucleotide sequence encoding a cleavage site for a protease or a portion of a protease not endogenously expressed by said cell, and (iii) a nucleotide sequence which encodes one of (y) a first inactive portion of a protein, which completes a second, inactive portion of a protein to produce a complete protein which produces a direct or indirect signal, or (z) a protein or portion of a protein which upon release from the fusion protein can be tracked as it moves, redistributes, translocates, or otherwise changes position within the cell, such as via targeted movement to a specific organelle and (b) a nucleic acid molecule which encodes a second fusion protein, the nucleic acid molecule comprising, (i) a nucleotide sequence which encodes a second test protein whose interaction with said first test protein in the present of said test compound is to be measured, and (ii) a nucleotide sequence which encodes a protease or a portion of a protease which is specific for the cleavage site in the first fusion protein, and determining a signal resulting from release of (a)(iii) from (a)(i) and (a)(ii) as a determination of modulation of said protein/protein interaction by said compound. Fluorescent proteins (*e.g.*, which produce a distinct color) are examples of proteins that can be tracked.

The first test protein may be a membrane bound protein, such as a transmembrane receptor, *e.g.*, a GPCR. Particular transmembrane receptors include, but are not limited to a β-adrenergic receptor (ADRB2), an arginine vasopressin receptor 2 (AVPR2), a serotonin receptor 1a (HTR1A), an m2 muscarinic acetylcholine receptor (CHRM2), a chemokine (C-C motif) receptor 5 (CCR5), a dopamine D2 receptor (DRD2), a kappa opioid receptor (OPRK), or an α1a-adregenic receptor (ADRA1A), although it is to be understood that in all cases the invention is not limited to these specific embodiments. For example, molecules such as the insulin growth factor-1 receptor (IGF-1R), which is a tyrosine kinase, and proteins which are not normally membrane bound, like estrogen receptor 1(ESR1) and estrogen receptors 2 (ESR2) can be utilized in the invention. A protease or portion of a protease may be a tobacco etch virus nuclear inclusion A protease. The first, inactive portion of the protein, when this embodiment is used, may be an inactive portion of an enzyme, or an inactive portion of a directly determinable protein, such as a fluorescent protein including those discussed herein. See, *e.g.* Cabantous, et al., Nature Biotechnology, 23:102-107 (2005), Hu et al. Mol Cell 9:789-98 (2002), and Ghosh et al. J Am Chem Soc 122:5658-5659 (2000). A protein or protein portion which produces a detectable signal, when this embodiment is used, may be a fluorescent protein, or any protein which upon movement within the cell, can be measured directly or indirectly. The second protein may be an inhibitory protein, such as an arrestin. The cell may be a eukaryote or a prokaryote. A reporter gene may be an exogenous gene, such as β-galactosidase, p-lactamase (Bla) or luciferase.

The nucleotide sequence encoding the first test protein may be modified to increase interaction with the second test protein. Such modifications include but are not limited to replacing all or part of the nucleotide sequence of the C-terminal region of the first test protein with a nucleotide sequence which encodes an amino acid sequence which has higher affinity for the second test protein than the original sequence. For example, the C-terminal region may be replaced by a nucleotide sequence encoding the C-terminal region of AVPR2, AGTRLI, GRPR, F2RL1, CXCR2/IL-8b, CCR4, or GRPR.

Some methods of the invention may comprise contacting more than one test compound to a plurality of samples of cells, each of said samples being contacted by one or more of the test compounds, wherein each of the cell samples have been transformed or transfected with any of the aforementioned nucleic acid molecules, and determining activity of reporter genes in the plurality of the samples to determine if any of the test compounds modulate a specific, protein/protein interaction. Some methods may comprise contacting each of the samples with one test compound, each of which differs from all others, or comprise contacting each of the samples with a mixture of the test compounds.

In yet another embodiment, there is provided a recombinant cell, transformed or transfected with (a) and (b) as described *supra*. One or both of the nucleic acid molecules may be stably incorporated into the genome of the cell. The cell also may have be or have been transformed or transfected with a reporter gene, when this embodiment is used. A first test protein may be a membrane bound protein, such as a transmembrane receptor, a GPCR, a cellular receptor, an ion channel, a growth factor receptor, or a cytokine receptor. Particular transmembrane receptors include, but are not limited to, ADRB2, AVPR2, HTR1A, CHRM2, CCR5, DRD2, OPRK, or AORA1A.

A protease or portion of a protease may be a tobacco etch virus nuclear inclusion A protease. When the first construct contains (y), i.e., a first, inactive portion of a protein which combines with a second, inactive portion of a protein to crease a functioning protein, it may be a portion of an enzyme, or a portion of a directly determinable protein, like a fluorescent protein. When the protein or protein portion provides a detectable signal, via movement within the cell, as elaborated upon *infra, i.e.*, when the construct contains (z), it may encode a fluorescent protein or protein portion, or any other protein or protein portion whose movement may be monitored. The second protein may be an inhibitory protein. The cell may be a eukaryote or a prokaryote. The reporter gene may be an exogenous gene, such as β-galactosidase, luciferase, aequorin, nitroreductase, beta glucourinidase, beta-lactamase, or alkaline phosphatase. Similarly, when embodiment (y) is used, this may be an inactive portion of aequorin, nitroreductase, β-galactosidase, luciferase, β-glucourinidose, β-lactamase, alkaline phosphatase, or any other enzyme known to the art. In the embodiment wherein (z) is used, *e.g.*, a nucleic acid molecule encoding a protein which generates a detectable signal, this may be a directly determinable protein, such as a fluorescent protein, exemplified by a green fluorescent protein, a red fluorescent protein, a cyan fluorescent protein, or a yellow fluorescent protein, or any protein or protein portion which can be measured.

Exemplary of such proteins are either whole proteins or epitopic portions of proteins which, upon movement through a cell, interact with labeled binding partners, such as fluorescent or halogen labeled binding partners, producing a detectable signal. Antigen antibody interaction is discussed herein, and the construct that is a feature of the invention may encode the antibody or the epitope or a whole, antigenic protein. The nucleotide sequence encoding the first test protein may be modified to increase interaction with the second test protein, such as by replacing all or part of the nucleotide sequence of the C-terminal region of the first test protein with a nucleotide sequence which encodes an amino acid sequence which has higher affinity for the second test protein than the original sequence. The C-terminal region may be replaced by a nucleotide sequence encoding the C-terminal region of AVPR2, AGTRLI, GRPR, F2RL1, CXCR2/IL-8B, CCR4, or GRPR.

In yet another embodiment, there is provided a test kit useful for determining if a test compound modulates a specific protein/protein interaction of interest comprising a separate portion of each of the nucleic acid molecule constructs (a) and (b) described *supra* and herein.

The first test protein may be a membrane bound protein, such as a transmembrane receptor. A particular type of transmembrane receptor is a GPCR. A particular transmembrane protein is a GPCR. Particular transmembrane receptors include ADRB2, AVPP2, HTR1A, CHRM2, CCR5, DRD2, OPRK, or ADRA1A. A protease or portion of a protease may be tobacco etch virus nuclear inclusion A protease. The second protein may be an inhibitory protein, such as an arrestin. A nucleotide sequence encoding the first test protein may be modified to increase interaction with the second test protein, such as by replacing all or part of the nucleotide sequence of the C-terminal region of the first test protein with a nucleotide sequence which encodes an amino acid sequence which has higher affinity for the second test protein than the original sequence. The nucleotide sequence of the C-terminal region may be replaced by a nucleotide sequence encoding the C-terminal region of AVPR2, AGTRLI, GRPR, F2RL1, CXCR2/IL-8B, CCR4, or GRPR

It is contemplated that any method or composition described herein can be implemented with respect to any other methods or composition described herein. The use of the word "a" or "an" when used in conjunction with the term "comprising" in the claims and/or the specification may mean "one," but it is also consistent with the meaning of "one or more," "at least one," and "one or more than one."

These, and other, embodiments of the invention will be better appreciated and understood when considered in conjunction with the following description and the accompanying drawings.

### BRIEF DESCRIPTION OF THE FIGURES

The following drawings form part of the present specification and are included to further demonstrate certain aspects of the present invention. The invention may be better understood by reference to one or more of these drawings in combination with the detailed description of specific embodiments presented herein.

Figure 1 shows the conceptual underpinnings of the invention, pictorially, using ligand-receptor binding as an example.

Figures 2a and 2b show that the response of targets in assays in accordance with the invention is dose dependent, both for agonists and antagonists.

Figure 3 shows that a dose response curve results with a different target and a different agonist as well.

Figure 4 depicts results obtained in accordance with the invention, using the D2 dopamine receptor.

Figures 5a and 5b illustrate results of an assay which shows that two molecules can be studied simultaneously.

Figure 6 sets forth the result of another "multiplex" assay, i.e., one where two molecules are studied simultaneously.

Figure 7 presents data obtained from assays measuring EGFR activity.

Figure 8 presents data obtained from assays in accordance with the invention, designed to measure the activity of human type I interferon receptor.

Figure 9 elaborates on the results in figure 7, showing a dose response curve for IFN-α in the cells used to generate figure 7.

Figure 10 shows the results of additional experiments where a different transcription factor and a different cell line were used.

### DETAINED DESCRIPTION OF THE INVENTION

The present invention relates to methods for determining if a substance of interest modulates interaction of a first test protein, such as a membrane bound protein, like a receptor, *e.g.*, a transmembrane receptor, with a second test protein, like a member of the arrestin family. The methodology involves cotransforming or cotransfecting a cell, which may be prokaryotic or eukaryotic, with two constructs. A first construct includes, a sequence encoding (i) the first test protein, such as a transmembrane receptor, (ii) a cleavage site for a protease not endogenously expressed by said cell, and (iii) a sequence encoding one of (y) a first, inactive portion of a protein, which upon release completes a second, inactive portion of a protein to produce a complete protein which produces a direct or indirect signal, and (z) a protein or protein portion which produces a detectable signal via its movement within a cell. A second construct includes, (i) a sequence which encodes a second test protein whose interaction with the first test protein is measured and/or determined, and (ii) a nucleotide sequence which encodes a protease or a portion of a protease specific for said cleavage site that is part of the first construct. In some embodiments, these constructs become stably integrated into the cells.

Additionally, the invention provides a first test protein comprising a recognition and cleavage site for a protease and a detectable peptide/polypeptide. In some embodiments, a detectable peptide/polypeptide is cleaved from a first test protein by a protease that recognize/cleaves at the recognition and cleavage site. In some embodiments, the detectable peptide/polypeptide can be cleaved from the first test protein by a protease that is associated with (*e.g.*, fused with) a second test protein. In some embodiments, the first test protein can be membrane bound, not membrane bound, in a cell, in an animal, outside of an animal, *in vivo*, *ex vivo* and/or *in vitro.*

The detectable peptide/polypeptide (DPP) can be detected by direct or indirect means. For example, a DPP can be a fluorescent protein wherein its fluorescence is different than when it is part of the fusion protein as compared to when cleaved. For example fluorescence of the polypeptide can increase or decrease when cleaved. In some embodiments, a DPP is an enzyme that is detectable, *e.g.*, using a substrate wherein the substrate and/or product are detectable. In some embodiments, a DPP can be a protein (*e.g.*, an enzyme such as Bla) wherein its activity changes when cleaved from a fusion protein. For example, the activity can increase or decrease upon cleavage from the fusion protein. In some embodiments, a DPP is a member of a protein fragment complementation pair. In this embodiment, the DPP is cleaved from the first test protein and is then "free" to interact with the second member of the protein fragment complementation pair to produce reporter protein, *e.g.*, an enzyme or a fluorescent protein.

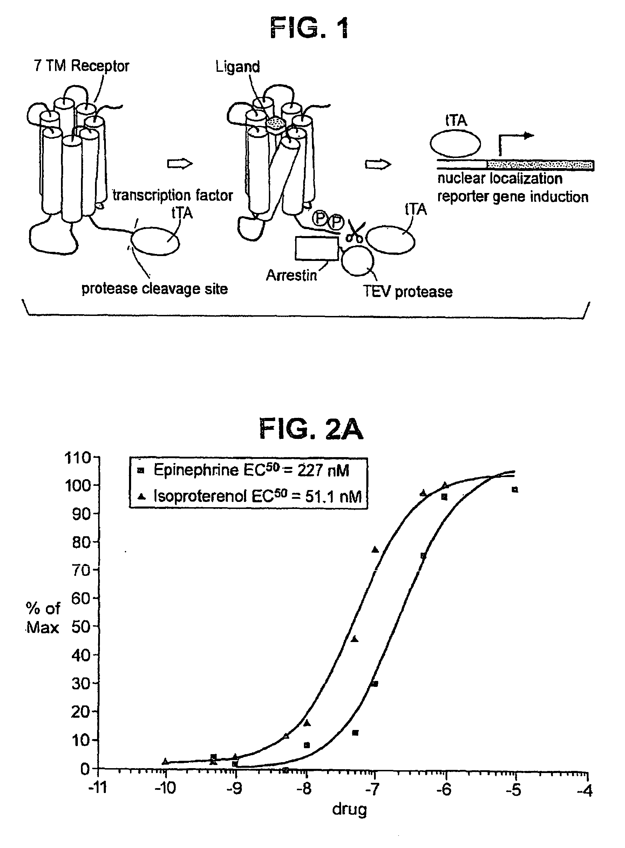

Some features of comparative method are shown, pictorially, in Figure 1. In brief, first, standard techniques are employed to fuse DNA encoding a transcription factor to DNA encoding a first test protein, such as a transmembrane receptor molecule, being studied. This fusion is accompanied by the inclusion of a recognition and cleavage site for a protease, *e.g.*, not expressed or expressed at relatively low levels endogenously by the host cell being used in the experiments. DNA encoding this first fusion protein is introduced into and is expressed by a cell which also contains a reporter gene sequence, under the control of a promoter element which is dependent upon the transcription factor fused to the first test protein, *e.g.*, the receptor. If the exogenous protease is not present and/or does not come in close proximity to the first test protein, the transcription factor remains tethered to the first test protein and is unable to enter the nucleus to stimulate expression of the reporter gene.

Recombinant techniques can also be used to produce a second fusion protein. For example in the comparative method depicted in Figure 1 , DNA encoding a member of the arrestin family is fused to a DNA molecule encoding a protease (*e.g.*, exogenous), resulting in a second fusion protein containing the second test protein, *e.g.*, an arrestin family member.

An assay is then carried out wherein the second fusion protein is expressed, together with the first fusion protein, and a test compound is contacted to the cells, *e.g.*, for a specific length of time. If the test compound modulates interaction of the two test proteins, *e.g.*, by stimulating, promoting or enhancing the association of the first and second test proteins, this can *e.g.*, lead to release of a DPP (*e.g.*, a transcription factor, which in turn moves to the nucleus, and provokes expression of the reporter gene). The activity of the reporter gene can be measured.

In an alternative system, the two test proteins may interact in the absence of a test compound, and the test compound may cause the two test proteins to dissociate, lessen or inhibit their interaction. In such a case, the level of free, functionally active transcription factor in the cell decreases in the presence of the test compound, leading to a decrease in proteolysis, and a measurable decrease in the activity of the reporter gene.

In the comparative method depicted in Figure 1, the arrestin protein, which is the second test protein, binds to the receptor in the presence of an agonist; however, it is to be understood that since receptors are but one type of protein, the assay is not dependent upon the use of receptor molecules, nor is agonist binding the only interaction capable of being involved. Any protein will suffice, although the interest in transmembrane proteins is clear. Further, agonist binding to a receptor is not the only type of binding which can be assayed. One can determine antagonists, *per se* and also determine the relative strengths of different antagonists and/or agonists in accordance with the disclosure.

When embodiment (y) of the first construct is used, the first, inactive portion of the protein, upon release, completes a second, inactive portion of a protein within the cell, resulting in a complete, active protein which generates a signal directly (e.g., by fluorescence), or indirectly (*e.g.*, by forming an active enzyme) which then acts upon a substrate to generate a signal, such as a color or fluorescence, or a change in color or fluorescence.

When embodiment (z) of construct (a) is used, the detectable protein changes location within the cell, and by determining its destruction, translocation, or relocalization, such as a specific organelle, one can determine or detect the protein-protein interaction being studied. A specific example of how translocation of a portion of a protein may be measured, can be seen in Wehrman, et al., Nature Methods, 2:521-527 (2005). Other systems will be known to the skilled artisan.

### I. Expression Constructs and Transformation

The term "vector" is used to refer to a carrier nucleic acid molecule into which a nucleic acid sequence can be inserted for introduction into a cell where it can be replicated. A nucleic acid sequence can be "exogenous," which means that it is foreign to the cell into which the vector is being introduced or that the sequence is homologous to a sequence in the cell but in a position within the host cell nucleic acid in which the sequence is ordinarily not found. Vectors include plasmids, cosmids, viruses (bacteriophage, animal viruses, and plant viruses), and artificial chromosomes (*e.g.*, YACs). One of skill in the art would be well equipped to construct a vector through standard recombinant techniques (see, for example, Maniatis, et al., Molecular Cloning, A Laboratory Manual (Cold Spring Harbor, 1990) and Ausubel, et al., 1994, Current Protocols In Molecular Biology (John Wiley & Sons, 1996) .

The term "expression vector" refers to any type of genetic construct comprising a nucleic acid coding for a RNA capable of being transcribed. In some cases, RNA molecules are then translated into a protein, polypeptide, or peptide. In other cases, these sequences are not translated, for example, in the production of antisense molecules or ribozymes. Expression vectors can contain a variety of "control sequences," which refer to nucleic acid sequences necessary for the transcription and possibly translation of an operably linked coding sequence in a particular host cell. In addition to control sequences that govern transcription and translation, vectors and expression vectors may contain nucleotide-sequences that serve other functions as well and are described *infra.*

1n certain embodiments, a plasmid vector is contemplated for use in cloning and gene transfer. In general, plasmid vectors containing replicon and control sequences which are derived from species compatible with the host cell are used in connection with these hosts. The vector ordinarily carries a replication site, as well as marking sequences which are capable of providing phenotypic selection in transformed cells. In a non-limiting example, *E. coli* is often transformed using derivatives of pBR322, a plasmid derived from an *E. coli* species. pBR322 contains genes for ampicillin and tetracycline resistance and thus provides easy means for identifying transformed cells. The pBR plasmid, or other microbial plasmid or phage must also contain, or be modified to contain, for example, promoters which can be used by the microbial organism for expression of its own proteins.

In addition, phage vectors containing replicon and control sequences that are compatible with the host microorganism can be used as transforming vectors in connection with these hosts. For example, the phage lambda GEM^{™}-11 may be utilized in making a recombinant phage vector which can be used to transform host cells, such as, for example, E. *coli* LE392.

Bacterial host cells, for example, *E. coli*, comprising the expression vector, are grown in any of a number of suitable media, for example, LB. The expression of the recombinant protein in certain vectors may be induced, as would be understood by those of skill in the art, by contacting a host cell with an agent specific for certain promoters, *e.g.*, by adding IPTG to the media or by switching incubation to a higher temperature. After culturing the bacteria for a further period, generally of between 2 and 24 h, the cells are collected by centrifugation and washed to remove residual media.

Many prokaryotic vectors can also be used to transform eukaryotic host cells. However, it may be desirable to select vectors that have been modified for the specific purpose of expressing proteins in eukaryotic host cells. Expression systems have been designed for regulated and/or high level expression in such cells. For example, the insect cell/baculovirus system can produce a high level of protein expression of a heterologous nucleic acid segment, such as described in U.S. Patents 5,871,986 and 4,879,236 , and which can be bought, for example, under the name MAXBAC^{®} 2.0 from INVITROGEN^{®} and BACPACK^{™} BACULOVIRUS EXPRESSION SYSTEM FROM CLONTECH^{®}.

Other examples of expression systems include STRATAGEN^{®}'s COMPLETE CONTROLS^{™} Inducible Mammalian Expression System, which involves a synthetic ecdysone-inducible receptor, or its pET Expression System, an *E.coli* expression system. Another example of an inducible expression system is available from INVITROGEN^{®}, which carries the T-REX™ (tetracycline-regulated expression) System, an inducible mammalian expression system that uses the full-length CMV promoter. INVITROGEN^{®} also provides a yeast expression system called the *Pichia methanolica* Expression System, which is designed for high-level production of recombinant proteins in the methylotrophic yeast *Pichia methanolica.* One of skill in the art would know how to express a vector, such as an expression construct, to produce a nucleic acid sequence or its cognate polypeptide, protein, or peptide.

### Regulatory Signals

The construct may contain additional 5' and/or 3' elements, such as promoters, poly A sequences, and so forth. The elements may be derived from the host cell, i.e., homologous to the host, or they may be derived from distinct source, i.e., heterologous.

A "promoter" is a control sequence that is a region of a nucleic acid sequence at which initiation and rate of transcription are controlled. It may contain genetic elements at which regulatory proteins and molecules may bind, such as RNA polymerase and other transcription factors, to initiate the specific transcription a nucleic acid sequence. The phrases "operatively positioned," "operatively linked," "under control," and "under transcriptional control" mean that a promoter is in a correct functional location and/or orientation in relation to a nucleic acid sequence to control transcriptional initiation and/or expression of that sequence.

A promoter generally comprises a sequence that functions to position the start site for RNA synthesis. The best known example of this is the TATA box, but in some promoters lacking a TATA box, such as, for example, the promoter for the mammalian terminal deoxynucleotidyl transferase gene and the promoter for the SV40 late genes, a discrete element overlying the start site itself helps to fix the place of initiation. Additional promoter elements regulate the frequency of transcriptional initiation. Typically, these are located in the region 30-110 bp upstream of the start site, although a number of promoters have been shown to contain functional elements downstream of the start site as well. To bring a coding sequence "under the control of" a promoter, one positions the 5' end of the transcription initiation site of the transcriptional reading frame "downstream" of (*i.e.,* 3' of) the chosen promoter. The "upstream" promoter stimulates transcription of the DNA and promotes expression of the encoded RNA.

The spacing between promoter elements frequently is flexible, so that promoter function is preserved when elements are inverted or moved relative to one another. In the tk promoter, the spacing between promoter elements can be increased to 50 bp apart before activity begins to decline. Depending on the promoter, it appears that individual elements can function either cooperatively or independently to activate transcription. A promoter may or may not be used in conjunction with an "enhancer," which refers to a cis-acting regulatory sequence involved in the transcriptional activation of a nucleic acid sequence.

A promoter may be one naturally associated with a nucleic acid molecule, as may be obtained by isolating the 5' non-coding sequences located upstream of the coding segment and/or exon. Such a promoter can be referred to as "endogenous." Similarly, an enhancer may be one naturally associated with a nucleic acid molecule, located either downstream or upstream of that sequence. Alternatively, certain advantages will be gained by positioning the coding nucleic acid segment under the control of a recombinant or heterologous promoter, which refers to a promoter that is not normally associated with a nucleic acid molecule in its natural environment. A recombinant or heterologous enhancer refers also to an enhancer not normally associated with a nucleic acid molecule in its natural environment. Such promoters or enhancers may include promoters or enhancers of other genes, and promoters or enhancers isolated from any other virus, or prokaryotic or eukaryotic cell, and promoters or enhancers not "naturally occurring," *i.e*., containing different elements of different transcriptional regulatory regions, and/or mutations that alter expression. For example, promoters that are most commonly used in recombinant DNA construction include the β-lactamase (penicillinase), lactose and tryptophan (trp) promoter systems. In addition to producing nucleic acid sequences of promoters and enhancers synthetically, sequences may be produced using recombinant cloning and/or nucleic acid amplification technology, including PCR™, in connection with the compositions disclosed herein (see U.S. Patents Nos. 4,683,202 and 5,928,906. Furthermore, it is contemplated the control sequences that direct transcription and/or expression of sequences within non-nuclear organelles such as mitochondria, chloroplasts, and the like, can be employed as well.

Naturally, it will be important to employ a promoter and/or enhancer that effectively directs the expression of the DNA segment in the organelle, cell type, tissue, organ, or organism chosen for expression. Those of skill in the art of molecular biology generally know the use of promoters, enhancers, and cell type combinations for protein expression, (see, for example Sambrook, *et al.*, 1989. The promoters employed may be constitutive, tissue-specific, inducible, and/or useful under the appropriate conditions to direct high level expression of the introduced DNA segment, such as is advantageous in the large-scale production of recombinant proteins and/or peptides. The promoter may be heterologous or endogenous.

Additionally any promoter/enhancer combination (as per, for example, the Eukaryotic Promoter Data Base EPDB, www.epd.isb-sib.ch/) could also be used to drive expression. Use of a T3, T7 or SP6 cytoplasmic expression system is another possible embodiment. Eukaryotic cells can support cytoplasmic transcription from certain bacterial promoters if the appropriate bacterial polymerase is provided, either as part of the delivery complex or as an additional genetic expression construct.

A specific initiation signal also may be required for efficient translation of coding sequences. These signals include the ATG initiation codon or adjacent sequences. Exogenous translational control signals, including the ATG initiation codon, may need to be provided. One of ordinary skill in the art would readily be capable of determining this and providing the necessary signals. It is well known that the initiation codon must be "in-frame" with the reading frame of the desired coding sequence to ensure translation of the entire insert. The exogenous translational control signals and initiation codons can be either natural or synthetic. The efficiency of expression may be enhanced by the inclusion of appropriate transcription enhancer elements.

In certain embodiments of the invention, the use of internal ribosome entry sites (IRES) elements are used to create multigene, or polycistronic, messages. IRES elements are able to bypass the ribosome scanning model of 5' methylated Cap dependent translation and begin translation at internal sites (Pelletier and Sonenberg, Nature, 334:320-325 (1988)). IRES elements from two members of the picornavirus family (polio and encephalomyocarditis) have been described (Pelletier and Sonenberg, *supra*), as well an IRES from a mammalian message (Macejak and Sarnow, Nature, 353:90-94 (1991)). IRES elements can be linked to heterologous open reading frames. Multiple open reading frames can be transcribed together, each separated by an IRES, creating polycistronic messages. By virtue of the IRES element, each open reading frame is accessible to ribosomes for efficient translation. Multiple genes can be efficiently expressed using a single promoter/enhancer to transcribe a single message (see U.S. Patent Nos. 5,925,565 and 5,935,819

### Other Vector Sequence Elements

Vectors can include a multiple cloning site (MCS), which is a nucleic acid region that contains multiple restriction enzyme sites, any of which can be used in conjunction with standard recombinant technology to digest the vector (see, for example, Carbonelli, et al, FEMS Microbial. Lett., 172(1):75-82 (1999), Levenson, et al., Hum. Gene Ther. 9(8):1233-1236 (1998), and Cocea, Biotechniques, 23(5):814-816 (1997)) "Restriction enzyme digestion" refers to catalytic cleavage of a nucleic acid molecule with an enzyme that functions only at specific locations in a nucleic acid molecule. Many of these restriction enzymes are commercially available. Use of such enzymes is widely understood by those of skill in the art. Frequently, a vector is linearized or fragmented using a restriction enzyme that cuts within the MCS to enable exogenous sequences to be ligated to the vector. "Ligation" refers to the process of forming phosphodiester bonds between two nucleic acid fragments, which may or may not be contiguous with each other. Techniques involving restriction enzymes and ligation reactions are well known to those of skill in the art of recombinant technology.

Most transcribed eukaryotic RNA molecules will undergo RNA splicing to remove introns from the primary transcripts. Vectors containing genomic eukaryotic sequences may require donor and/or acceptor splicing sites to ensure proper processing of the transcript for protein expression (see, for example, Chandler, *et al.*, 1997).

The vectors or constructs of the present invention will generally comprise at least one termination signal. A "termination signal" or "terminator" comprises a DNA sequence involved in specific termination of an RNA transcript by an RNA polymerase. Thus, in certain embodiments a termination signal that ends the production of an RNA transcript is contemplated. A terminator may be necessary *in vivo* to achieve desirable message levels.

In eukaryotic systems, the terminator region may also comprise specific DNA sequences that permit site-specific cleavage of the new transcript so as to expose a polyadenylation site. This signals a specialized endogenous polymerase to add a stretch of about 200 adenosine residues (polyA) to the 3' end of the transcript. RNA molecules modified with this polyA tail appear to more stable and are translated more efficiently. Thus, in some embodiments involving eukaryotes, a terminator may comprise a signal for the cleavage of the RNA. In some embodiments, a terminator signal promotes polyadenylation of the message. In some embodiments, terminator and/or polyadenylation site elements can serve to enhance message levels and to minimize read through from the cassette into other sequences.

Terminators contemplated for use in the invention include any known terminator of transcription described herein or known to one of ordinary skill in the art, including but not being limited to, for example, the termination sequences of genes, such as the bovine growth hormone terminator, viral termination sequences, such as the SV40 terminator. In certain embodiments, the termination signal may be a lack of transcribable or translatable sequence, such as an untranslatable/untranscribable sequence due to a sequence truncation.

In expression, particularly eukaryotic expression, one will typically include a polyadenylation signal to effect proper polyadenylation of the transcript. The nature of the polyadenylation signal is not believed to be crucial to the successful practice of the invention, and any such sequence may be employed. Some embodiments include the SV40 polyadenylation signal or the bovine growth hormone polyadenylation signal, both of which are convenient, readily available, and known to function well in various target cells. Polyadenylation may increase the stability of the transcript or may facilitate cytoplasmic transport.

In order to propagate a vector in a host cell, it may contain one or more origins of replication sites (often termed "ori"), sites, which are specific nucleotide sequences at which replication is initiated. Alternatively, an autonomously replicating sequence (ARS) can be employed if the host cell is yeast.

### Transformation Methodology

Suitable methods for nucleic acid delivery for use with the current invention are believed to include virtually any method by which a nucleic acid molecule (*e.g.*, DNA) can be introduced into a cell as described herein or as would be known to one of ordinary skill in the art. Such methods include, but are not limited to, direct delivery of DNA such as by ex vivo transfection (Wilson, et al., Science, 244:1344-1346 (1989), Nabel et at, Science, 244:1342-1344 (1989), by injection (U.S. Patent Nos. 5,994,624, 5,981,274, 5,945,100, 5,780,448, 5,736,524, 5,702,932, 5,656,610, 5,589,466 and 5,580,859, including microinjection (Harlan and Weintraub, J. Cell Biol., 101(3):1094-1099 (1985); U.S. Patent No. 5,789,215; by electroporation (U.S. Patent No. 5,384,253; Tur-Kaspa, et al., Mol: Cell Biol., 6:716-718 (1986); Potter, et al., Proc. Natl. Acad Sci. USA, 81:7161-7165 (1984); by calcium phosphate precipitation (Graham and Van Der Eb, Virology, 52:456-467 (1973); Chen and Okayama, Mol. Cell Biol., 7(8):2745-2752 (1987); Rippe, et al., Mol. Cell Biol., 10:689-695 (1990); by using DEAE-dextran followed by polyethylene glycol (Gopal, Mol. Cell Biol., 5:1188-190 (1985); by direct sonic loading (Fechheimer, et al., Proc. Natl. Acad Sci. USA, 89(17):8463-8467 (1987); by liposome mediated transfection (Nicolau and Sene, Biochem. & Biophys. Acta., 721:185-190 (1982); Fraley, et al., Proc. Natl. Acad. Sci. USA, 76:3348-3352 (1979); Nicolau, et al., Meth. Enzym., 149:157-176 (1987); Wong, et al., Gene, 10:879-894 (1980); Kaneda, et al., Science, 243:375-378 (1989); Kato, et al., .J. Biol. Chem., 266:3361-3364 (1991) and receptor-mediated transfection (Wu and Wu, J. Biol. Chem., 262:4429-4432 (1987); Wu and Wu, J Biol Chem. 263(29):14621-4. (1988); by PEG-mediated transformation of protoplasts (Omirulleh, et al., Plant Mol. Biol., 21(3):415-428 (1987); U.S. Patent Nos. 4,684,61 and 4,952,500 ; by desiccation/inhibition-mediated DNA uptake (Potrykus, et al. Mol. Gen. Genet., 199(2):169-177 (1985), and any combination of such methods.

### II. Components of the Assay System

As with the methods described herein, the products which are features of the invention have various embodiments. Some embodiments of the invention provide a "three part construct," *e.g.*, that contains sequences encoding a test protein, a cleavage site, and a DPP. In some embodiments, a test protein is a membrane bound protein, such as a transmembrane receptor, *e.g.*, a member of the GPCR family. In some embodiments, these sequences can be modified so that the C-terminus of the protein they encode has better and stronger interactions with the second protein. The modifications can include, *e.g.*, replacing a C-terminal encoding sequence of the test protein, such as a GPCR, with the C terminal coding region for AVPR2, AGTRLI, GRPR, F2PLI, CCR4, CXCR2/IL-8, CCR4, or GRPR, examples of which are described herein.

In an embodiment which uses an inactive portion of a protein, this is chosen so that, upon release from the construct, *e.g.*, a fusion protein, it combines with a second, also inactive portion of a protein, to create a complete, active protein (protein complementation). This complete, active protein either generates a detectable signal, such as by luminescence or fluorescence, or an indirect signal, such as by forming a complete enzyme which then acts upon a substrate to provide a detectable signal.

In an embodiment where a detectable protein is used, this is chosen such that, upon release from, *e.g.*, a fusion protein, the detectable protein can be tracked as it moves, redistributes, translocates, or otherwise changes position within the cell, such as via targeted movement to a specific organelle. In some embodiments, a fluorescent proteins produce a distinct color.

The second construct, as described herein, includes a region which encodes a protein that interacts with the first protein, leading to some measurable phenomenon. In some embodiments, a protein may be an activator, an inhibitor, or, more, generically, a "modulator" of the first protein. In some embodiments, members of the arrestin family are utilized, *e.g.*, when the first protein is a GPCR, but other protein encoding sequences may be used, *e.g.*, when the first protein is not a GPCR. A second part of these two part constructs encodes a protease, or portion of a protease, which acts to remove a DPP from a fusion protein encoded by the first construct.

However, these embodiments do not limit the invention, as discussed in the following additional embodiments.

### Host Cells

As used herein, the terms "cell," "cell line," and "cell culture" may be used interchangeably. All of these terms also include their progeny, which is any and all subsequent generations. It is understood that all progeny may not be identical due to deliberate or inadvertent mutations. The host cells generally will have been engineered to express a screenable or selectable marker which is activated by the transcription factor that is part of a fusion protein, along with the first test protein.

In the context of expressing a heterologous nucleic acid sequence, "host cell" refers to a prokaryotic or eukaryotic cell that is capable of replicating a vector and/or expressing a heterologous gene encoded by a vector. When host cells are "transfected" or "transformed" with nucleic acid molecules, they are referred to as "engineered" or "recombinant" cells or host cells, *e.g.*, a cell into which an exogenous nucleic acid sequence, such as, for example, a vector, has been introduced. Therefore, recombinant cells are distinguishable from naturally-occurring cells which do not contain a recombinantly introduced nucleic acid.

Numerous cell lines and cultures are available for use as a host cell, and they can be obtained through the American Type Culture Collection (ATCC), which is an organization that serves as an archive for living cultures and genetic materials (www.atcc.org). An appropriate host can be determined by one of skill in the art based on the vector backbone and the desired result. A plasmid or cosmid, for example, can be introduced into a prokaryote host cell for replication of many vectors. Cell types available for vector replication and/or expression include, but are not limited to, bacteria, such as *E. coli* (*e.g.*, *E. coli* strain RR1, *E. coli* LE392, *E. coli* B, *E. coli* X 1776 (ATCC No. 31537) as well as *E. coli* W3110 (F-, lambda-, prototrophic, ATCC No. 273325), DH5a, JM109, and KC8, bacilli such as *Bacillus subtilis*; and other enterobacteriaceae such as *Salmonella typhimurium, Serratia marcescens,* various *Pseudomonas* specie, as well as a number of commercially available bacterial hosts such as SURE^{®} Competent Cells and SOLOPACK^{™} Gold Cells (STRATAGEN^{®}, La Jolla). In certain embodiments, bacterial cells such as *E. coli* LE392 are particularly contemplated as host cells for phage viruses.

Examples of cukaryotic host cells for replication and/or expression of a vector include, but are not limited to, HeLa, NIH3T3, Jurkat, 293, COS, CHO, Saos, PerC6, CHO and PC 12 cells and derivatives thereof. Many host cells from various cell types and organisms are available and would be known to one of skill in the art. Similarly, a viral vector may be used in conjunction with either a eukaryotic or prokaryotic host cell, particularly one that is permissive for replication or expression of the vector.

### Test Proteins

The present invention contemplates the use of any two or more proteins for which a physical interaction is known, suspected or desired to be investigated. The two proteins will exist as fusions proteins, *e.g.*, a first test protein fused to a DPP (*e.g.*, a transcription factor), and the second test protein fused to a protease that recognizes a cleavage site in the first fusion protein, cleavage of which releases the DPP, the directly or indirectly determinable protein or the inactive portion of a protein.

With respect to a first construct, a first test protein may be, *e.g.*, a naturally membrane bound protein, or one which has been engineered to become membrane bound, via standard techniques. The first test protein may be, *e.g.*, a transmembrane receptor such as any of the GPCRs, or any other transmembrane receptor of interest, including, but not being limited to, receptor tyrosine kinases, receptor serine threonine kinases, cytokine receptors, and so forth. In some embodiments, a first and/or second test protein is a cellular receptor, an ion channel, a growth factor receptor, a receptor tyrosine kinase, a Toll-like receptor (TLR; *e.g.*, TLR1, TLR2, TLR3, TLR4, TLR5, TLR6, TLR7, TLR8, TLR9, TLR10, TLR11, TLR12, and TLR13), NF-kappaB signaling pathway proteins (*e.g.*, NF-κB1 (also called p50), NF-κB2 (also called p52), RelA (also named p65), RelB, IκB kinase and c-Rel), a cytokine receptor, an integrin, a cell adhesion protein or an estrogen receptor and other nuclear hormone/steroid hormone receptor superfamily members. In some embodiments, a first and/or second test protein is a ligand, *e.g.*, for a receptor as described herein. In some embodiments, a first and/or second test protein is a thyroid hormone receptor-like or a ligand thereof such as a Group A: thyroid hormone receptor (Thyroid hormone), *e.g.*, a thyroid hormone receptor-α (TRa; NR1A1) or a thyroid hormone receptor-β (TRβ; NR1A2); a Group B: Retinoic acid receptor (*e.g.*, Vitamin A and related compounds), *e.g.*, a retinoic acid receptor-α (RARα; NR1B1), a retinoic acid receptor-β (RARβ; NR1B2) and a retinoic acid rcceptor-γ (RARγ; NR1B3); a Group C: Peroxisome proliferator-activated receptor, *e.g.*, a peroxisome proliferator-activated receptor-α (PPARα; NR1C1), a peroxisome proliferator-activated receptor-β (PPARβ; NR1C2), and a peroxisome proliferator-activated receptor-γ (PPARγ: NR1C3); a Group D: Rev-erb, *e.g.*, a Rev-erbα (Rev-erbα; NR1D1) and a Rev-erbβ (Rev-erbβ; NR1D2); a Group F: Retinoid-related orphan receptor, *e.g.*, a retinoid-related orphan receptor-α (RORα; NR1F1), a retinoid-related orphan receptor-β (RORβ; NR1F2) and a retinoid-related orphan receptor-γ (RORγ; NR1F3); a Group H: Liver X receptor-like, *e.g.*, a liver X receptor-α (LXRα; NR1H3), a liver X receptor-β (LXRβ; NR1H2), and a famesoid X receptor (FXR; NR1H4); and a Group I: Vitamin D receptor-like, *e.g.*, a vitamin D receptor (VDR; NR1II), a pregnane X receptor (PXR; NR1I2), and a constitutive androstane receptor (CAR; NR1I3). In some embodiments, a first and/or second test protein is a retinoid X receptor-like or a ligand thereof such as a Group A: Hepatocyte nuclear factor-4 (HNF4), *e.g.*, a hepatocyte nuclear factor-4-α (HNF4α NR2A1) and a hepatocyte nuclear factor-4-y (HNF4γ; NR2A2); a Group B: Retinoid X receptor (RXRα), *e.g.*, a retinoid X receptor-α (RXRα; NR2B1), a retinoid X receptor-β (RXRβ; NR2B2), and a retinoid X receptor-γ (RXRγ; NR2B3); a Group C: Testicular receptor, *e.g.*, a testicular receptor 2 (TR2; NR2C1) and a testicular receptor 4 (TR4; NR2C2); a Group E: TLX/PNR, *e.g.*, a human homologue of the Drosophila tailless gene (TLX; NR2E1) and a Photoreceptor-Specific Nuclear Receptor (PNR; NR2E3); and a Group F: COUP/EAR, *e.g.*, a Chicken ovalbumin upstream promoter-transcription factor I (COUP-TFI; NR2F1), a Chicken ovalbumin upstream promoter-transcription factor II (COUP-TFII; NR2F2) and a ERBA-related 2 (EAR2; NR2F6). In some embodiments, a first and/or second test protein is an Estrogen Receptor-like (Steroid hormone receptor) or a ligand thereof such as a Group A: Estrogen receptor (Sex hormone receptors; sex hormones: Estrogen), *e.g.*, an Estrogen receptor-α (ERa; NR3A1) and an Estrogen receptor-β (ERβ; NR3A2); a Group B: Estrogen related receptor, *e.g.*, an Estrogen related receptor-α (ERRα; NR3B1), an Estrogen related receptor-β (ERRβ; NR3B2) and an Estrogen related receptor-γ (ERRγ; NR3B3); and a Group C: 3-Ketosteroid receptors, *e.g.*, a Glucocorticoid receptor (GR; NR3C1) (Cortisol), a Mineralocorticoid receptor (MR; NR3C2) (Aldosterone), a Progesterone receptor (PR; NR3C3) (*e.g.*, Sex hormone receptor; Sex hormones: Progesterone) and an Androgen receptor (AR; NR3C4) (*e.g.*, Sex hormone receptors; Sex hormones: Testosterone). In some embodiments, a first and/or second test protein is a Nerve Growth Factor IB-like or a ligand thereof such as a Group A: NGFIB/NURR1/NOR1, *e.g.*, a Nerve Growth factor IB (NGFIB; NR4A1), a Nuclear receptor related 1 (NURR1; NR4A2) and a Neuron-derived orphan receptor I (NOR1; NR4A3). In some embodiments, a first and/or second test protein is a Steroidogenic Factor-like or a ligand thereof such as a Group A: SF1/LRH1, *e.g.,* a Steroidogenic factor 1 (SF1; NRSA1) and a Liver receptor homolog 1 (LRH1; NR5A2). In some embodiments, a first and/or second test protein is a Germ Cell Nuclear Factor-like or a ligand thereof such as a GCN1, *e.g.,* a Germ cell nuclear factor (GCN1; NR6A1).

Further, as it is well known that portions of proteins will function in the same manner as the full length first test protein, such active portions of a first test protein are encompassed by the definition of protein herein.

As will be evident to the skilled artisan, the present invention may be used to assay for interaction with any protein, and is not limited in its scope to assaying membrane bound receptor, like the GPCRs. For example, the activity of other classes of transmembrane receptors, including but not limited to: receptor tyrosine kinases (RTKs), such as IGF1R, such as the epidermal growth factor receptor (EGFR), ErbB2/HER2/Neu or related RTKs; receptor serine/threonine kinases, such as Transforming Growth Factor-beta (TGFβ), activin, or Bone Morphogenetic Protein (BMP) receptors; cytokine receptors, such as receptors for the interferon family for interleukin, erythropoietin, G-CSF, GM-CSF, tumor necrosis factor (TNF) and leptin receptors; and other receptors, which are not necessarily normally membrane bound, such as estrogen receptor 1 (ESR1), and estrogen receptor 2 (ESR2). Some methods of the invention involve transfecting a cell with a modified receptor construct that directs the expression of a chimeric protein containing the receptor of interest (or a portion thereof), to which is appended, a protease cleavage site and a DPP. In some embodiments, a cell is co-transfected with a second construct that directs the expression of a chimeric protein consisting of an interacting protein fused, to the protease that recognizes and cleaves the site, *e.g.,* as described herein. In the case of RTKs, such as the EGFR, this interacting protein may consist of a SH2 (Src homology domain 2) containing a protein or portion thereof, such as phospholipase C (PLC) or Src homology 2 domain containing transforming protein 1 (SHC1). In the case of receptor serine/threonine kinases, such as TGFβ, activin, BMP receptors, this interacting protein may be a Smad protein or portion thereof. In the case of cytokine receptors, such as interferon-α/β or interferon-γ gamma receptors, this interacting protein may be a signal transducer and activator of transcription (STAT) protein such as, but not being limited to, Stat1, Stat2; Janus kinase (JAK) proteins Jak1, Jak2, or Tyk2; or portions thereof.

Other possible test protein pairs include, but are not limited to, antibody-ligands, enzyme-substrates, kinases and phosphorylation substrates, phosphatases and phosphorylated proteins, dimerizing proteins, components of signal transduction cascades, and other protein pairs well known to the art.

In an embodiment where position of protein within a cell is determined, the protein is chosen such that, upon release from, *e.g.,* a fusion protein, it can be tracked as it moves, redistributes, translocates, or otherwise changes position without the cell, such as via targeted movement to a specific organelle. Some embodiments of the invention utilize fluorescent proteins which produce a distinct color, but other protein and protein portions, such as antibody epitope interactions, or enzymatic action on substrates (*e.g.,* specifically located), can be used.

### Reporters and DPPs

Reporters and DDPs are proteins that can directly or indirectly result in a detectable signal. In some embodiments, a DPP can be directly detected, *e.g.,* increases or decreases fluorescence. In some embodiments, a DPP can be "split" into two polypeptides, *e.g.,* wherein the DPP displays higher activity when the split two polypeptides are associated with each other. Many split DPPs are known in the art, *e.g.* β-galactosidase (*e.g.,* see Johnson and Varshavsky, PNAS 91:10340-44 (1994) and U.S. Patent No. 7,166,424), Dihydrofolate reductase (*e.g.,* see Pelletier et al., PNAS 95:12141-46; Remy and Michnick, PNAS 96:5394-99; and U.S. Patent No. 6,872,871), fluorescent protein such as GFP and variants (*e.g*., see Hu et al. Mol Cell 9:789-98 (2002); Ghosh et al. J Am Chem Soc 122:5658-5659 (2000); and U.S. Patent Nos. 7,166,424), a protease such as a tobacco etch virus (TEV) protease *(e.g.,* see Wehr et al. Nature Methods 3(12):985-993 (2006) and U.S. Patent Publication No. 20050084864), Bla (*e.g.,* see Wehrman et al. Proc. Natl. Acad. Sci. USA 99: 3469-3474 (2002); Galarneau et al., Nat. Biotechnol. 20:619-622 (2002); Spotts et al. Proc. Natl. Acad. Sci. USA 99:15142-15147 (2002); and U.S. Patent No. 6,828,099), luciferase (*e.g.,* see Remy and Michnick, Nature Methods 3(12):977-979 (2006)), an intein such as a *Synechoytstis* dnaE intein (*e.g.,* see Ozawa et al., Anal. Chem. 73:2516-2521 (2001)), and a luciferase such as firefly, *Renilla,* or *G. princeps* (Remy and Michnick, Nat. Methods 3:977-979 (2006); Paulmurugan and Gambhir, Anal Chem 75:1584-1589 (2003); and Paulmurugan et al., Proc. Natl. Acad. Sci. USA 99: 5608-15613 (2002)). Also, *see e.g.,* Kerppola, Nature Methods 3(12): 969-971 (2006).

In some cases a protease can be considered as a DPP if its activity can be directly or indirectly detected, *e.g.,* cleavage of a protein by the protease causes an increase or decrease in a detectable signal. Some embodiments of the invention, exploit a protease to activate or deactivate secondary reporters.

In the embodiment of the invention using a protein which modulates a reporter gene, this may be any protein having an impact on a gene, expression or lack thereof which leads to a detectable change in gene expression. Typical protein reporters include enzymes such as chloramphenicol acetyl transferase (CAT), β-glucuronidase (GUS) β-galactosidase, nitroreductase, aequorin, luciferase, beta lactamase, and alkaline phosphatase. Also contemplated are fluorescent and chemilluminescent proteins such as a green fluorescent protein, a red fluorescent protein, a cyan fluorescent protein, and a yellow fluorescent protein.

A variety of assays have been constructed based either on activity of wild-type beta-galactosidase or on the phenomenon of alpha- or omega-complementation. Beta-gal is a multimeric enzyme which forms tetramers and octomeric complexes of up to 1 million Daltons. Beta-gal subunits undergo self-oligomerization which leads to activity. This naturally-occurring phenomenon has been used to develop a variety of in vitro, homogeneous assays. Alpha- or omega-complementation of beta-gal has been utilized to develop assays for the detection of antibody-antigen, drug-protein, protein-protein, and other bio-molecular interactions. However, the adaptation of beta-gal complementation to live cell assays has been limited because the phenomenon occurs naturally, resulting in significant background activity. The background activity problem has been overcome in part by the development of low-affinity, mutant subunits with a diminished or negligible ability to complement naturally, enabling various assays including for example the detection of ligand-dependent activation of the EGF receptor in live cells. In some embodiments, beta-gal is expressed with a localization signal, e.g., a nuclear localization signal.

Luminescent, fluorescent or bioluminescent signals are easily detected and quantified with any one of a variety of automated and/or high-throughput instrumentation systems including fluorescence multi-well plate readers, fluorescence activated cell sorters (FACS) and automated cell-based imaging systems that provide spatial resolution of the signal. A variety of instrumentation systems have been developed to automate high content screening (HCS) including the automated fluorescence imaging and automated microscopy systems developed by Cellomics (now part of Thermo-Fisher), Amersham (now part of GE), TTP, Q3DM (now part of Beckman Coulter), Evotec (now part of Perkin-Elmer), Universal Imaging (now part of Molecular Devices) and Zeiss. Fluorescence recovery after photobleaching (FRAP) and time lapse fluorescence microscopy have also been used to study protein mobility in living cells. The optical instrumentation and hardware have advanced to the point that essentially any bioluminescent signal can be detected with high sensitivity and high throughput

### Transcriptions Factors and Repressors

A transcription factor or other DPP can be used to activate expression of a reporter gene in an engineered host cell. Transcription factors are typically classified according to the structure of their DNA-binding domain, which are generally (a) zinc fingers, (b) helix-tum-helix, (c) leucine zipper, (d) helix-loop-helix, or (e) high mobility groups. The activator domains of transcription factors interact with the components of the transcriptional apparatus (RNA polymerase) and with other regulatory proteins, thereby affecting the efficiency of DNA binding.

The Rel/Nuclear Factor kB (NF-kB) and Activating Protein-1 (AP-1) are among the most studied transcription factor families. They have been identified as important components of signal transduction pathways leading to pathological outcomes such as inflammation and tumorogenesis. Other transcription factor families include the heat shock/E2F family, POU family and the ATF family. A tTA or GAL4 are transcription factors.

Though transcription factors are one class of molecules that can be used, the assays of the present disclosure may be modified to accept the use of transcriptional repressor molecules, where the measurable signal is downregulation of a signal generator, or even cell death.

In an embodiment of the invention which uses an inactive portion of a protein (*e.g.,* protein complementation), this is chosen so that, upon release from the construct, *e.g.,* a portion of a fusion protein, it combines with a second, also inactive portion of a protein, to create a complete, active protein. This complete, active protein either generates a detectable signal, such as by luminescence, or an indirect signal, such as by forming a complete enzyme which then acts upon a substrate to provide a detectable signal. In some embodiments, the two inactive portions of a protein are from a transcription factor

### Proteases and Cleavage Sites

Proteases are well characterized enzymes that cleave other proteins at a particular site. One family, the Ser/Thr proteases, cleaves at serine and threonine residues. Other proteases include cysteine or thiol proteases, aspartic proteases, metalloproteinases, aminopeptidases, di & tripeptidases, carboxypeptidases, and peptidyl peptidases. The choice of these is left to the skilled artisan and certainly need not be limited to the molecules described herein. It is well known that enzymes have catalytic domains and these can be used in place of full length proteases. Such are encompassed by the invention as well. A specific embodiment is the tobacco etch virus nuclear inclusion A protease, or an active portion thereof. Other specific cleavage sites for proteases may also be used, as will be clear to the skilled artisan.

### Modification of Test Proteins

A first test protein may be modified to enhance its binding to an interacting protein (*e.g.,* a second test protein) in this assay. For example, it is known that certain GPCRs bind arrestins more stably or with greater affinity upon ligand stimulation and this enhanced interaction is mediated by discrete domains, *e.g.,* clusters of serine and threonine residues in the C-terminal tail (Oakley, et al., J. Biol. Chem., 274:32248-32257, 1999 and Oakley, et al., J. Bio/. Chem., 276:19452-19460, 2001). Using this as an example, it is clear that the receptor encoding sequence itself may be modified, so as to increase the affinity of a membrane bound protein, such as the receptor, with a protein to which it binds. Exemplary of such modifications are modifications of the C-terminal region of a membrane bound protein, *e.g.,* a receptor, such as those described herein. Some embodiments involve replacing a portion of a protein with a corresponding region of another protein (*e.g.,* a receptor), which has higher affinity for the binding protein, but does not impact the receptor function. Examples 16 and 20, *supra,* show embodiments of this feature of the invention.

In addition, a second test protein may be modified to enhance its interaction with a first test protein. For example, the assay may incorporate point mutants, truncations or other variants of the second test protein, *e.g.,* arrestins that are known to bind agonist-occupied GPCRs more stably or in a phosphorylation-independent manner (Kovoor, et al., J. Biol. Chem., 274:6831-6834, 1999).

### III. Kits

Any of the compositions described herein may be comprised in a kit. The kits will thus comprise, in suitable container means for the vectors or cells of the present invention, and any additional agents that can be used in accordance with the present invention.

The kits may comprise a suitably aliquoted compositions of the present invention. The components of the kits may be packaged either in aqueous media or in lyophilized form. The container means of the kits will generally include at least one vial, test tube, flask, bottle, syringe or other container means, into which a component may be placed, and optionally, suitably aliquoted. Where there are more than one component in the kit, the kit also will generally contain a second, third or other additional container into which the additional components may be separately placed. However, various combinations of components may be comprised in a vial. The kits of the present invention also will typically include a means for containing reagent containers in close confinement for commercial sale. Such containers may include injection or blow-molded plastic containers into which the desired vials are retained.

When the components of the kit are provided in one and/or more liquid solutions, the liquid solution may be an aqueous solution, *e.g.,* with a sterile aqueous solution. In some embodiments, components of a kit may be provided as dried powder(s). When reagents and/or components are provided as a dry powder, the powder can be reconstituted by the addition of a suitable solvent. It is envisioned that the solvent may also be provided in another container means.

### IV. Comparative Examples

The comparative examples which follow, do not form part of the invention, but form part of the present disclosure.

### COMPARATIVE EXAMPLE 1

A fusion construct was created, using DNA encoding human β2 adrenergic receptor, referred to hereafter as "ADRB2", in accordance with standard nomenclature. Its nucleotide sequence can be found at GenBank, under Accession Number NM_000024 (SEQ ID NO: 1). The tetracycline controlled transactivator tTA, described by Gossen, et al., Proc. Natl. Acad. Sci. USA, 87:5547-5551 (1992), incorporated by reference, was also used. A sequence encoding the recognition and cleavage site for tobacco etch virus nuclear inclusion A protease, described by Parks, et al., Anal. Biochem., 216:413-417 (1994), incorporated by reference, is inserted between these sequences in the fusion coding gene. The CMV promoter region was placed upstream of the ADRB2 coding region, and a poly A sequence was placed downstream of the tTA region.

A fusion construct was prepared by first generating a form of ADRB2 which lacked internal BamHI and BglII restriction sites. Further, the endogenous stop codon was replaced with a unique BamHI site.

Overlapping PCR was used to do this. To elaborate, a 5' portion of the coding region was amplified with:
gattgaagat ctgccttctt gctggc (SEQ ID NO: 2),
and
gcagaacttg gaagacctgc ggagtcc (SEQ ID NO: 3),
while a 3' portion of the coding region was amplified with:
ggactccgca ggtcttccaa gttctgc (SEQ ID NO: 4),
and
ttcggatcct agcagtgagt catttgt (SEQ ID NO: 5).

The resulting PCR products have 27 nucleotides of overlapping sequence and were purified via standard agarose gel electrophoresis. These were mixed together, and amplified with SEQ ID NO: 2, and SEQ ID NO: 5.

PCR was also used to modify the coding region of tTA so that the endogenous start codon was replaced with a TEV NIa-Pro cleavage site. The cleavage site, defined by the seven amino acid sequence ENLYFQS (SEQ ID NO: 6), is taught by Parks, et al., Anal. Biochem., 216:413-417 (1994), incorporated by reference. The seventh amino acid is known as P1' position, and replacing it with other amino acids is known to reduce the efficiency of cleavage by TEV NIa-Pro. See Kapust, et al., Biochem. Biophys. Res. Commun., 294:949-955 (2002).

Variants where the seventh amino acid was changed to Tyr, and where it was changed to Leu, were produced. These resulted in intermediate and low efficiency cleavage sites, as compared to the natural high efficiency site.

A DNA sequence encoding the natural high efficiency site was added to the tTA coding region in two steps. Briefly, BamHI and Xbal restriction sites were added to the 5' end and a XhoI restriction site was added to the 3' end of the tTA coding region by PCR with
ccggatcctc tagattagat aaaagtaaag tg (SEQ ID NO: 7)
and
gactcgagct agcagtatcc tcgcgccccc taccc (SEQ ID NO: 8),
and the TEV NIa-Pro cleavage site was added to the 5' end by ligating an oligonucleotide with the sequence
gagaacctgt acttccag (SEQ ID NO: 9)
between the BamHI and XbaI sites.

This DNA sequence was modified to encode the intermediate and low efficiency cleavage sites by PCR using:
ggatccgaga acctgtactt ccagtacaga tta (SEQ ID NO: 10),
and
ctcgagagat cctcgcgccc cctacccacc (SEQ ID NO: 11).
for ENLYFQY (SEQ ID NO: 12), and
ggatccgaga acctgtactt ccagctaaga tta (SEQ ID NO: 13),
and
ctcgagagat cctcgcgccc cctacccacc (SEQ ID NO: 11) for ENLYFQL (SEQ ID NO: 14).

These PCR steps also introduced a BamHI restriction site 5' to the sequence encoding each cleavage site, and an XhoI restriction site 3' to tTA stop codon.

The thus modified ADRB2 coding region was digested with PstI, which cuts at nucleotide position 260 in the coding region, and BamHI. This 3' fragment was ligated with the three variants of tTA modified with the TEV NIa-Pro cleavage sites, that had been digested with BamHI and Xhol, and the resulting complexes were cloned into pBlueScript II, which had been digested with PstI and XhoI.

A NotI restriction site was introduced 5' to the start codon of the ADRB2 coding region, again via PCR, using
gcggccgcca ccatgaacgg taccgaaggc cca (SEQ ID NO: 15),
and
ctggtgggtg gcccggtacc a (SEQ ID NO: 16).

The 5' fragment of modified ADRB2 coding region was isolated, via digestion with NotI and PstI and was ligated into each of the constructs of the 3' fragment of ADRB2-TEV-NIa-Pro-cleavage site tTA fusions that had been digested previously, to produce three, full length constructs encoding fusion proteins.

Each construct was digested with NotI and XhoI, and was then inserted into the commercially available expression vector pcDNA 3, digested with NotI and XhoI.

### COMPARATIVE EXAMPLE 2

A second construct was also made, whereby the coding sequence for "β arrestin 2 or ARRB2" hereafter (GenBank, NM_004313) (SEQ ID NO: 17), was ligated to the catalytic domain of the TEV NIa protease (i.e., amino acids 189-424 of mature NIa protease, residues 2040-2279) in the TEV protein. To do this, a DNA sequence encoding ARRB2 was modified, so as to add a BamHI restriction site to its 5' end. Further, the sequence was modified to replace the endogenous stop codon with a BamHI site. The oligonucleotides
caggatcctc tggaatgggg gagaaacccg ggacc (SEQ ID NO: 18),
and
ggatccgcag agttgatcat catagtcgtc (SEQ ID NO: 19)
were used. The resulting PCR product was cloned into the commercially available vector pGEM-T EASY (Promega). The multiple cloning site of the pGEM-T EASY vector includes an EcoRI site 5' to the start codon of ARRB2.

The TEV NIa-Pro coding region was then modified to replace the endogenous start codon with a BglII site, and to insert at the 3' end a sequence which encodes influenza hemagluttinin epitope YPYDVPDYA (SEQ ID NO: 20) in accordance with Kolodziej, et al., Meth. Enzymol., 194:508-519 (1991), followed by a stop codon, and a NotI restriction site. This was accomplished via PCR, using
agatctagct tgtttaaggg accacgtg (SEQ ID NO: 21),
and
gcggccgctc aagcgtaatc tggaacatca tatgggtacg agtacaccaa ttcattcatg ag (SEQ ID NO: 22).

The resulting, modified ARRB2 coding region was digested with EcoRI and BamHI, while the modified TEV coding region was cleaved with BgIII and NotI. Both fragments were ligated into a commercially available pcDNA3 expression vector, digested with EcoRI and Notl.

### COMPARATIVE EXAMPLE 3

Plasmids encoding ADRB2-TEV-NIa-Pro cleavage site-tTA and the ARRB2-TEV-NIa protease fusion proteins were transfected into HEK-293T cells, and into "clone 41," which is a derivative of HEK-293T, that has a stably integrated β-galactosidase gene under control of a tTA dependent promoter. About 5x10⁴ cells were plated in each well of a 24 well plate, in DMEM medium supplemented with 10% fetal bovine serum, 2mM *L*-glutamine, 100 units/ml penicillin, 100 µg/ml G418, and 5 µg/ml purimycin. Cells were grown to reach 50% confluency the next day, and were then transfected, using 0.4 µg plasmid DNA, and 2 µl Fugene (a proprietary transfection reagent containing lipids and other material). The mix was combined in 100 µl of DMEM medium, and incubated for 15 minutes at room temperature prior to adding cells. Transfected cells were incubated for 8-20 hours before testing by adding drugs which are known agonists for the receptor, and then 16-24 hours after drug addition.

### COMPARATIVE EXAMPLE 4

The levels of β-galactosidase activity in the cells were first measured by staining the cells with a chromogenic substance, i.e., "X-gal," as taught by MacGregor, et al., Somat. Cell Mol. Genet., 13:253-265 (1987), incorporated by reference. Following culture, cells were washed, twice, in D-PBS with calcium and magnesium, fixed for 5 minutes in 4% paraformaldehyde, and then washed two additional times with D-PBS, calcium and magnesium, for 10 minutes each time. Fixed cells were incubated with 5mM potassium ferricyanide, 5mM potassium ferrocyanide_{;} 2mM MgCl₂, 0.1% X-Gal, that had been prepared from a 1:40 dilution of 4% X-Gal stock in dimethylformamide, in D-PBS with calcium and magnesium.

The reaction was incubated in the dark at room temperature for from 3-4 hours, to overnight. Substrate solution was removed, and cells were mounted under glass coverslips with mowiol mounting medium (10% mowiol, 0.1% 1.4-diazabicyclo[2.2.2]octane, 24% glycerol).

The results indicated that cells transfected with either the ADRB2-TEV-NIa-Pro cleavage site-tTA plasmid alone or the ARRB2-TEV-NIa protease plasmid alone did not express β-galactosidase. A small fraction of cells transfected with both plasmids did express β-galactosidase, probably due to basal levels of interaction between unstimulated ADRB2 and ARRB2. About 3-5 fold more cells expressed the reporter gene after treatment with either 10 µM isoproterenol, or 10 µM epinephrine, both of which are ADRB2 agonists.

When the cells were pretreated for 5 minutes with the ADRB2 antagonist alprenolol (10 µM), the agonist induced increase in β-galactosidase expressing cells was blocked, and treatment with alprenolol alone had no apparent effect.

These results show that one can link agonist binding and GPCR stimulation to transcriptional activation of a reporter gene.

### COMPARATIVE EXAMPLE 5

A set of experiments were carried out in order to quantify the level of reporter gene activity in the cells more precisely and to maximize the signal-to-background ratio of the assay. This was accomplished by measuring the level of reporter gene induction using a commercially available chemiluminescence assay for β-galactosidase activity. Clone 41 cells were transfected with the ADRB2-tTA fusion constructs, containing either the high, medium or low efficiency cleavage sites, and the ARRB2-TEV-NIa protease expression plasmid described *supra.* Cells were either untreated or treated with 1 µM isoproterenol 20 hours after the transfection, and the luminescence assay was carried out 24 hours after the drug addition. In brief, following cell culture, the medium was removed, and 50 µl of lysis buffer (100 mM potassium phosphate, pH7.8, 0.2% Triton X-100) was added to each well. The cells were lysed via incubation for 5 minutes, at room temperature, with mild agitation. Lysates were collected and analyzed via commercially available products.

In all cases, treatment with agonist increased levels of β-galactosidase activity. However, the background level of reporter gene activity in untreated cells was lowest with the low efficiency cleavage site, relative to the medium and high efficiency sites. Further, agonist treatment resulted in a 4.8-fold stimulation of reporter gene activity in cells transfected with the low efficiency cleavage site, compared to 2.8-fold for the medium efficiency cleavage site and 1.2-fold for the high efficiency cleavage site. Thus, the highest signal-to-background ratio is obtained by using the low efficiency protease cleavage site.

### COMPARATIVE EXAMPLE 6

These experiments were designed to verify that the agonist stimulated increase in reporter gene expression is dependent on binding and activation of the receptor by the agonist.

To do this, variants of the ADRB2-tTA fusion constructs were generated following the protocols *supra,* except each contained a mutant form of the receptor with a single amino acid change from D to S at position 113, which results in a greatly reduced affinity for the agonist isoproterenol. See Strader, et al., J. Biol. Chem., 266:5-8 (1991). Three forms of the mutant reeeptor-tTA fusion construct with each of the different cleavage sites were formed.

The levels of β-galactosidase activity were measured in clone 41 cells co-transfected with the ADRB2-tTA fusion constructs containing the D113S point mutation and the ARRB2-TEV-NIa protease expression plasmid described previously. The activity tests were carried out exactly as described, *supra.* The results indicated that the agonist isoproterenol did not stimulate reporter gene expression in cells expressing the mutant ADRB2-tTA fusion contructs.

### COMPARATIVE EXAMPLE 7

These experiments were designed to examine whether the agonist stimulated increase in reporter gene expression is dependent on fusion of TEV NIa-Pro to ARRB2.

To do this, the levels of β-galactosidase activity were measured in clone 41 cells co-transfected with the ADRB2-tTA fusion construct containing the low efficiency cleavage site and either the ARRB2-TEV-NIa protease expression plasmid described *supra,* or a control TEV-NIa protease fusion to the SH2 domain of phospholipase C. The activity tests were carried out exactly as described, *supra.* The results indicated that agonist-stimulated increase in reporter gene expression was detected only when the TEV protease was fused to ARRB2 and not when fused to an unrelated polypeptide.

### COMPARATIVE EXAMPLE 8

These experiments were designed to determine if gene expression is induced selectively by agonists of the target receptor, or if it can be stimulated by other molecules.

ATP is an agonist for G protein coupled receptors P2Y1 and P2Y2, which are expressed endogenously by HEK-293T cells.

Experiments were carried out using clone 41 cells which were cotransfected with the ADRB2-tTA fusion construct containing the low efficiency cleavage site and the arrestin-TEV-NIa protease fusion as described *supra,* which were treated with isoproterenol, ATP, or untreated. The assays were carried out as described, *supra.*

The results indicated that induction of reporter gene activity was specific to activation of target receptor. Stimulation of another GPCR pathway was irrelevant.

### COMPARATIVE EXAMPLE 9

A set of experiments were carried out using clone 41 cells which were cotransfected with the ADRB2-tTA fusion construct containing the low efficiency cleavage site and the ARRB2-TEV-NIa protease fusion as described *supra,* which were treated with varying amounts of one of the adrenergic receptor agonists isoproterenol and epinephrine. The assays were carried out as described, *supra.* The results presented in figure 2a show a dose-response curve for the stimulation of reporter gene expression by these two ligands. Each point represents the mean value obtained from three experiments.

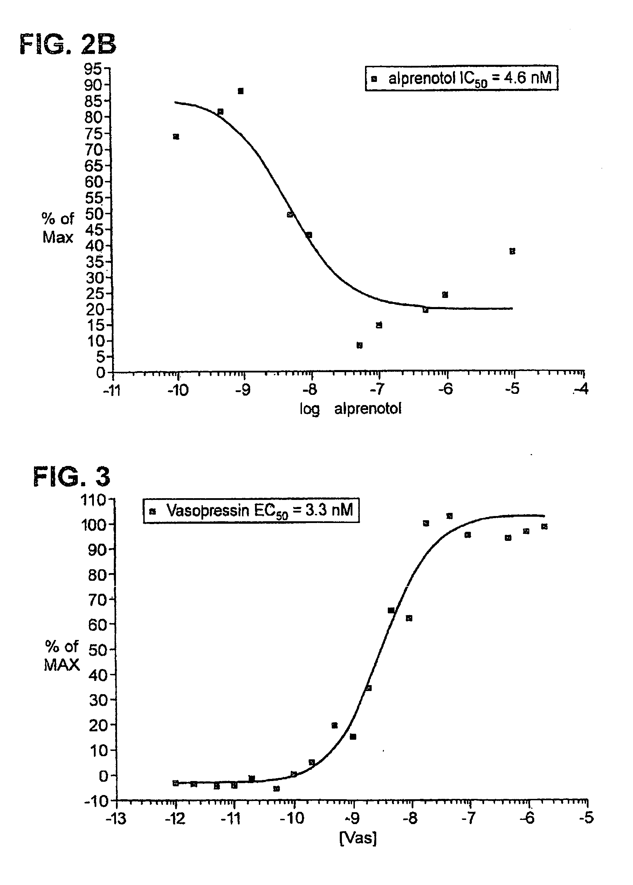

A set of experiments were carried out as described *supra,* in which the co-transfected clone 41 cells were pretreated with varying concentrations of the adrenergic receptor antagonist alprenolol for 15 minutes, followed by treatment with 1 :M epinephrine. The results shown in figure 2b indicate a dose-inhibition curve for this antagonist.

### COMPARATIVE EXAMPLE 10

A similar set of constructs were made to establish an assay for the G protein coupled arginine vasopressin receptor 2 (AVPR2). The AVPR2 coding region (Genbank Accession Number: NM_000054) (SEQ ID NO: 23) was modified to place an EcoRI site at the 5' end and replace the stop codon with a BamHI site using PCR with the primers
gaattcatgc tcatggcgtc caccac (SEQ ID NO: 24)
and
ggatcccgat gaagtgtcct tggccag (SEQ ID NO: 25).

The modified AVPR2 coding region was ligated into the three ADRB2- tTA constructs described *supra,* which had been cut with EcoRI and BamHI. This replaced the entire coding sequence of the ADRB2 with the coding sequence of AVPR2.

Clone 41 cells were co-transfected with the AVPR2-tTA fusion construct containing the low efficiency cleavage site and the ARRB2-TEV-NIa protease fusion described *supra,* and assays were carried out using varying concentrations (1 pM to 2 µM) of [Arg8] vasopressin, an agonist for AVPR2. The data, presented in figure 3, shows a dose-response curve for this agonist, with an EC50 of 3.3 nM, which agrees with previously published data (Oakley, R., et. al., Assay and Drug Development Technologies, 1:21-30, (2002)). The maximal response resulted in an approximately 40-fold induction of reporter gene expression over the background level.

### COMPARATIVE EXAMPLE 11

A similar set of constructs were made to establish an assay for the G protein coupled serotonin receptor 1a (HTR1A). The HTR1A coding region (Genbank Accession Number: NM_000524) (SEQ ID NO: 26) was modified to place an EcoRI site at the 5' end and replace the stop codon with a BamHI site using PCR with the primers
gaattcatgg atgtgctcag ccctgg (SEQ ID NO: 27)
and
ggatccctgg cggcagaact tacac (SEQ ID NO: 28).

The modified HTR1A coding region was ligated into the AVPR2- tTA constructs described *supra,* which had been cut with EcoRI and BamHI. This replaced the entire coding sequence of AVPR2 with the coding sequence of HTR1A. The resulting construct will be referred to as "HTR1A-tTA" hereafter.

Clone 41 cells were co-transfected with the HTR1A-tTA fusion construct containing the low efficiency cleavage site and the ARRB2-TEV-NIa protease fusion construct described *supra,* and assays were carried out using 10 µM 8-hydroxy-DPAT HBr (OH-DPAT), an agonist for the HTR1A, as well as with 10 µM serotonin, a natural agonist for HTR1A. The assays were carried out as described, *supra.* The maximal response to OH-DPAT resulted in a 6.3-fold induction of reporter gene expression over background level and the maximal response to serotonin resulted in a 4.6-fold induction of reporter gene expression over background level.

### COMPARATIVE EXAMPLE 12

Similar constructs were made to establish an assay for the G protein coupled m2 muscarinic acetylcholine receptor (CHRM2). The CHRM2 coding region (Genbank Accession Number: NM_000739) (SEQ ID NO: 29) was modified to place an EcoRI site at the 5' end and replace the stop codon with a BglII site using PCR with the primers
gaattcatga ataactcaac aaactcc (SEQ ID NO: 30)
and
agatctcctt gtagcgccta tgttc (SEQ ID NO: 31).

The modified CHRM2 coding region was ligated into the AVPR2 - tTA constructs described *supra,* which had been cut with EcoRI and BamHI. This replaced the entire coding sequence of AVPR2 with the coding sequence of CHRM2.

Clone 41 cells were co-transfected with the CHRM2-tTA fusion construct containing the high efficiency cleavage site and the ARRB2-TEV-NIa protease fusion described *supra,* where the ARRB2-protease fusion protein was expressed under the control of the Herpes Simplex Virus thymidine kinase (HSV-TK) promoter, and assays were carried out using 10 µM carbamylcholine Cl (carbochol), an agonist for CHRM2, as described *supra.* The maximal response to carbochol resulted in a 7.2-fold induction of reporter gene expression over background.

### COMPARATIVE EXAMPLE 13

a Constructs were also made to establish an assay for the G protein coupled chemokine (C-C motif) receptor 5 (CCR5). The CCR5 coding region (Genbank Accession Number: NM_000579) (SEQ ID NO: 32) was modified to place Not I site at the 5' end and replace the stop codon with a BamHI site using PCR with the primers
gcggccgcat ggattatcaa gtgtcaagtc c (SEQ ID NO: 33)
and
ggatccctgg cggcagaact tacac (SEQ ID NO: 34).

The CCR5 coding region was also modified to place a BsaI site at the 5' end which, when cut, leaves a nucleotide overhang which is compatible with EcoRI cut DNA using the primers
ggtctccaat tcatggatta tcaagtgtca agt (SEQ ID NO: 35)
and
gacgacagcc aggtacctat c (SEQ ID NO: 36).

The first modified coding region was cut with ClaI and BamHI and the second was cut with BsaI and ClaI. Both fragments were ligated into the AVPR2 - tTA constructs described *supra,* which had been cut with EcoRI and BamHI. This replaced the entire coding sequence of AVPR2 with the coding sequence of CCR5.

The CCR5-tTA fusion construct containing the low efficiency cleavage site was transfected into "clone 34" cells, which are a derivative of the HEK cell line "clone 41" described *supra,* but which contain a stably integrated ARRB2-TEV-NIa protease fusion gene under the control of the CMV promoter. Assays were carried out using 1 µg/ml "Regulated on Activation, Normal T-Cell Expressed and Secreted" (RANTES), a known agonist for CCR5. The maximal response to RANTES, measured as described *supra* resulted in an approximately 40-fold induction of reporter gene expression over the background.

### COMPARATIVE EXAMPLE 14

Next, a set of constructs were made to establish an assay for the G protein coupled dopamine 2 receptor (DRD2). The DRD2 coding region (Genbank Accession Number: NM_000795) (SEQ ID NO: 37) was modified to place an EcoRI site at the 5' end and replace the stop codon with a BglII site using PCR with the primers
gaattcatgg atccactgaa tctgtcc (SEQ ID NO: 38)
and
agatctgcag tggaggatct tcagg (SEQ ID NO: 39).

The modified DRD2 coding region was ligated into the AVPR2 - tTA constructs described *supra,* cut with EcoRI and BamHI. This replaced the entire coding sequence of AVPR2 with the coding sequence of DRD2.

Clone 41 cells were co-transfected with the DRD2-tTA fusion construct containing the medium efficiency cleavage site and the ARRB2-TEV-NIa protease fusion described *supra,* and assays were carried out using 10 µM dopamine HCl (dopamine), an agonist for DRD2. Results were measured as in the assays described *supra.* The maximal response to dopamine resulted in a 2.7-fold induction of reporter gene expression over the background.

### COMPARATIVE EXAMPLE 15

These experiments were designed to demonstrate enhancements of the assay using arrestin variants that bind agonist-occupied GPCRs more stably. First, a fusion of the TEV NIa protease to β-arrestin-1 (ARRB1) was constructed. The coding region of ARRB1 (Genbank Accession Number: NM_004041) (SEQ ID NO: 40) was modified to place an Asp718 site at the 5' end and replace the stop codon with a BamHI site using PCR with the primers
ggtaccatgg gcgacaaagg gacgcgagtg (SEQ ID NO: 41)
and
ggatcctctg ttgttgagct gtggagagcc tgtaccatcc tcctcttc (SEQ ID NO: 42).

The resulting modified ARRB 1 coding region was cut with Asp718 and EcoRI and with EcoRI and BamHI, while the modified TEV NIa-Pro coding region described *supra* was cut with BglII and NotI. All three fragments were ligated into a commercially available pcDNA3 expression vector, which had digested with Asp718 and NotI.

Clone 41 cells were co-transfected with the DRD2-tTA fusion construct containing the medium efficiency cleavage site and the ARRB1-TEV-NIa protease fusion, and assays were carried out using 10 µM dopamine HCl (dopamine), an agonist for the D2 receptor, as described *supra.* The maximal response to dopamine resulted in a 2.1-fold induction of reporter gene expression over the background.

Truncation of ARRB1 following amino acid 382 has been reported to result in enhanced affinity for agonist-bound GPCRs, independent of GRK-mediated phosphorylation (Kovoor A., et. al., J. Biol. Chem., 274(11):6831-6834 (1999)). To demonstrate the use of such a "constitutively active" arrestin in the present assay, the coding region of β-arrestin-1 was modified to place an Asp718 site at the 5' end and a BamHI site after amino acid 382 using PCR with SEQ ID NO: 41, *supra*
and
ggatccattt gtgtcaagtt ctatgag (SEQ ID NO: 43).

This results in a an ARRb1 coding region which is 36 amino acids shorter than the full-length coding region. The resulting modified ARRB1 coding region, termed "ARAB1 (Δ383)", was cut with Asp718 and EcoRI and with EcoRI and BamHI, while the modified TEV NIa-Pro coding region described *supra* was cut with BgIII and NotI. All three fragments were ligated into a commercially available pcDNA3 expression vector, digested with Asp718 and NotI.

Clone 41 cells were co-transfected with the DRD2-tTA fusion construct containing the medium efficiency cleavage site and the ARRB1 (Δ383)-TEV-NIa protease fusion, and assays were carried out using 10 µM dopamine HCl (dopamine), an agonist for the DRD2 receptor, as described *supra.* The maximal response to dopamine resulted in an 8.3-fold induction of reporter gene expression over the background.

To examine the effect of a comparable truncation of the ARRB2 coding region the coding region of ARRB2 was modified to place an Asp718 site at the 5' end and replaced 81 nucleotides at the 3' end with a BamHI site using PCR with the primers
ggtaccatgg gggagaaacc cgggacc (SEQ ID NO: 44)
and
ggatcctgtg gcatagttgg tatc (SEQ ID NO: 45).

This results in a ARRB2 coding region which is 27 amino acids shorter than the full-length coding region. The resulting modified ARRB2 coding region was cut with Asp718 and BamHI, while the modified TEV NIa-Pro coding region described *supra* was cut with BglII and NotI. Both fragments were ligated into a commercially available pcDNA3 expression vector, digested with Asp718 and NotI.

Clone 41 cells were co-transfected with the DRD2-tTA fusion construct containing the medium efficiency cleavage site and the ARRB22 (Δ383)-TEV-NIa protease fusion, and assays were carried out using 10 µM dopamine HCl (dopamine), an agonist for the DRD2 receptor, as described *supra.* The maximal response to dopamine resulted in a 2.1-fold induction of reporter gene expression over the background.

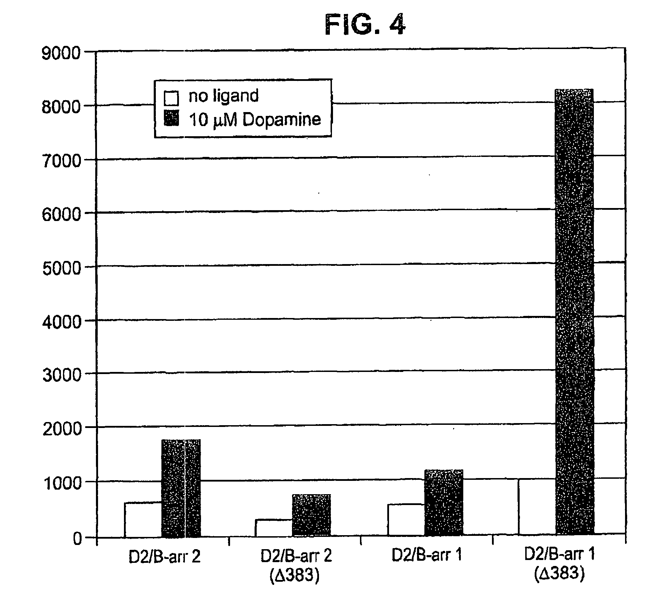

These results, presented in figure 4, demonstrate that DRD2 dopamine receptor assay shows the highest signal-to-background ratio using the arrestin variant ARRB1 (Δ383).

### COMPARATIVE EXAMPLE 16

This set of experiments was carried out to demonstrate enhancements of the assay using receptor modifications that are designed to increase affinity for the interacting protein. In this example, the C-terminal tail domain of a test receptor was replaced with the corresponding tail domain from AVPR2, a receptor known to bind arrestins with high affinity. In these examples the fusion junction was made 15-18 amino acids after the conserved NPXXY motif at the end of the seventh transmembrane helix, which typically corresponds to a position immediately after a putative palmitoylation site in the receptor C-terminus.

First, PCR was used to produce a DNA fragment encoding the C-terminal 29 amino acids from AVPR2, followed by the low efficiency TEV cleavage site and tTA transcription factor. The fragment was also designed such that the first two amino acids (Ala, A and Arg, R) are encoded by the BssHII restriction site GCGCGC. This was accomplished by amplifying the AVPR2-tTA construct with the low efficiency cleavage site described *supra,* with the primers
tgtgcgcgcg gacgcacccc acccagcctg ggt (SEQ ID NO: 46)
and
ctcgagagat cctcgcgccc cctacccacc (SEQ ID NO: 11).

Next, the coding region of the DRD2 was modified to place an EcoRI site at the 5' end and to insert a BssHII site after the last amino acid in the coding region (Cys-443). This was done using PCR with the primers
gaattcatgg atccactgaa tctgtcc (SEQ ID NO: 47)
and
tgtgcgcgcg cagtggagga tcttcaggaa ggc (SEQ ID NO: 48).

The resulting modified D2 coding region was cut with EcoRI and BssHII and the resulting AVPR2 C-terminal tail-low efficiency cleavage site-tTA fragment was cut with BssHII and BamHI. Both fragments were ligated into the AVPR2-low efficiency cleavage site-tTA construct described *supra,* cut with EcoRI and BamHI.

Clone 41 cells were co-transfected with the DRD2-AVPR2 Tail-tTA fusion construct containing the low efficiency TEV cleavage site and the ARRB2-TEV-NIa protease fusion described *supra,* and assays were carried out using 10 µM dopamine HCl (dopamine), an agonist for the DRD2 receptor. The maximal response to dopamine resulted in an approximately 60-fold induction of reporter gene expression over the background.

A construct was made which modified the ADRB2 receptor coding region by inserting an Asp718 site at the 5' end and by placing a BssHII site after Cys-341. This was done using PCR with the primers
gcggccgcca ccatgaacgg taccgaaggc cca (SEQ ID NO: 49)
and
tgtgcgcgcg cacagaagct cctggaaggc (SEQ ID NO: 50).

The modified ADRB2 receptor coding region was cut with EcoRI and BssHII and the AVPR2 C-terminal tail-low efficiency cleavage site-tTA fragment was cut with BssHII and BamHI. Both fragments were ligated into the AVPR2-low efficiency cleavage site-tTA construct described *supra* cut, with EcoRI and BamHI. The resulting construct is "ADRB2-AVPR2 Tail-tTA." (Also see published application U.S. 2002/0106379, *supra,* SEQ ID NO: 3 in particular.)

Clone 41 cells were co-transfected with the ADRB2-AVPR2 Tail-tTA fusion construct containing the low efficiency TEV cleavage site and the ARRB2-TEV-NIa protease fusion described *supra,* and assays were carried out using 10 µM isoproterenol, an agonist for the ADRB2 receptor. The maximal response to isoproterenol resulted in an approximately 10-fold induction of reporter gene expression over the background.

A construct was made which modified the kappa opioid receptor (OPRK; Genbank Accession Number: NM_000912) (SEQ ID NO: 51) coding region by placing a BssHII site after Cys-345. This was done using PCR with the primers
ggtctacttg atgaattcct ggcc (SEQ ID NO: 52)
and
gcgcgcacag aagtcccgga aacaccg (SEQ ID NO: 53)

The modified OPRK receptor coding region was cut with EcoRI and BssHII and AVPR2 C-terminal tail-low efficiency cleavage site-tTA fragment was cut with BssHII and Xhol. Both fragments were ligated into a plasmid containing the modified OPRK receptor sequence, cloned into pcDNA3.1+ at Asp718 (5') and XhoI (3'), which had been digested with EcoRI and XhoI.

Clone 41 cells were co-transfected with the OPRK-AVPR2 Tail-tTA fusion construct containing the low efficiency cleavage site and the ARRB2-TEV-NIa protease fusion described *supra,* and assays were carried out using 10 µM U-69593, an agonist for the OPRK. The maximal response to U-69593 resulted in an approximately 12-fold induction of reporter gene expression over the background.

### COMPARATIVE EXAMPLE 17

This experiment was designed to demonstrate the use of the assay to measure the activity of two test receptors simultaneously using a multiplex format.

Clone 41 cells and "clone 1H10" cells, which are cells of an HEK-293T cell line containing a stable integration of the luciferase gene under the control of a tTA-dependent promoter, were each plated on 24-well culture dishes and were transiently transfected with the chimeric ADRB2-AVPR2 Tail-tTA or the DRD2-AVPR2 Tail-tTA fusion constructs described *supra,* respectively. Transient transfections were performed using 100µl of media, 0.4µg of DNA and 2µl of FuGene reagent per well. After 24 hr of incubation, Clone 41 cells expressing ADRB2-AVPR2 Tail-tTA and clone 1H10 cells expressing DRD2-AVPR2 Tail-tTA were trypsinized, mixed in equal amounts, and replated in 12 wells of a 96-well plate. Triplicate wells were incubated without drug addition or were immediately treated with 1µM isoproterenol, 1µM dopamine, or a mixture of both agonists at 1µM. Cells were assayed for reporter gene activity approximately 24 hours after ligand addition. Medium was discarded, cells were lysed in 40µl lysis buffer [100 mM potassium phosphate pH 7.8, 0.2% Triton X-100] and the cell lysate was assayed for beta-galactosidase and for luciferase activity using commercially available luminescent detection reagents.

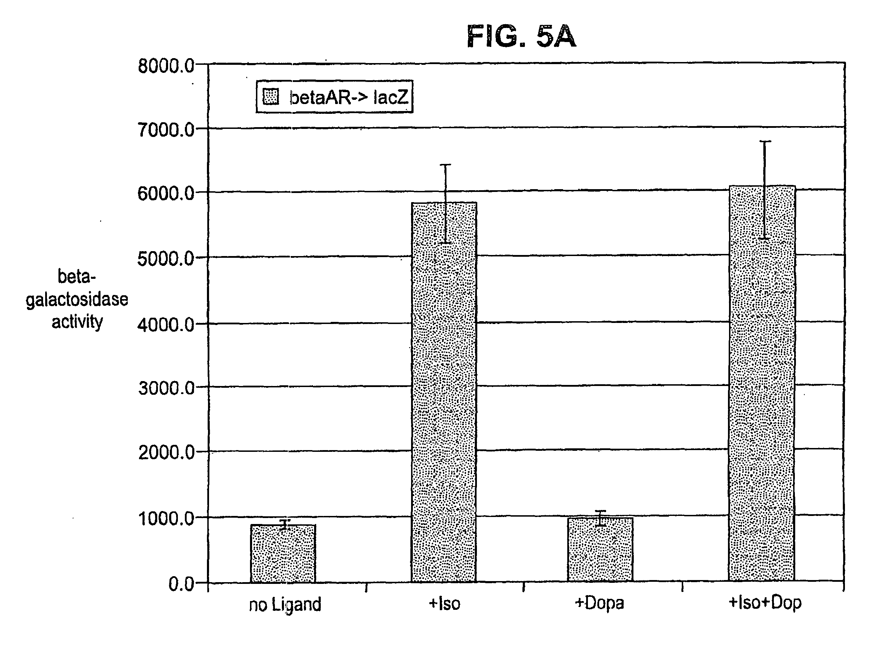

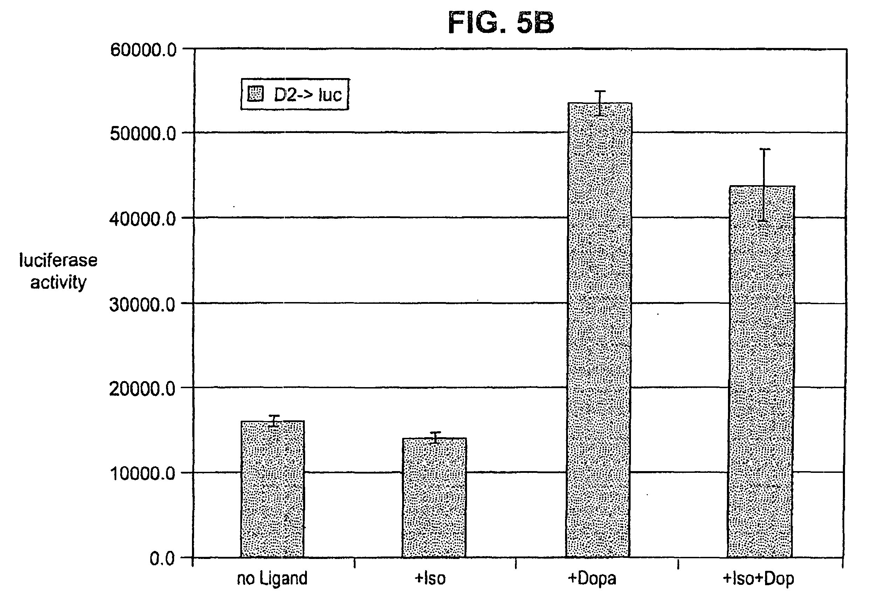

The results are presented in Figure 5A and SB. Treatment with isoproterenol resulted in an approximately seven-fold induction of beta-galactosidase reporter gene activity, whereas luciferase activity remained unchanged. Treatment with dopamine resulted in a 3.5-fold induction of luciferase activity, while beta-galactosidase activity remained unchanged. Treatment with both isoproterenol and dopamine resulted in seven-fold and three-fold induction of beta-galactosidase and luciferase activity, respectively.

### COMPARATIVE EXAMPLE 18

This experiment was designed to demonstrate the use of the assay to measure the activity of two test receptors simultaneously using a multiplex format.

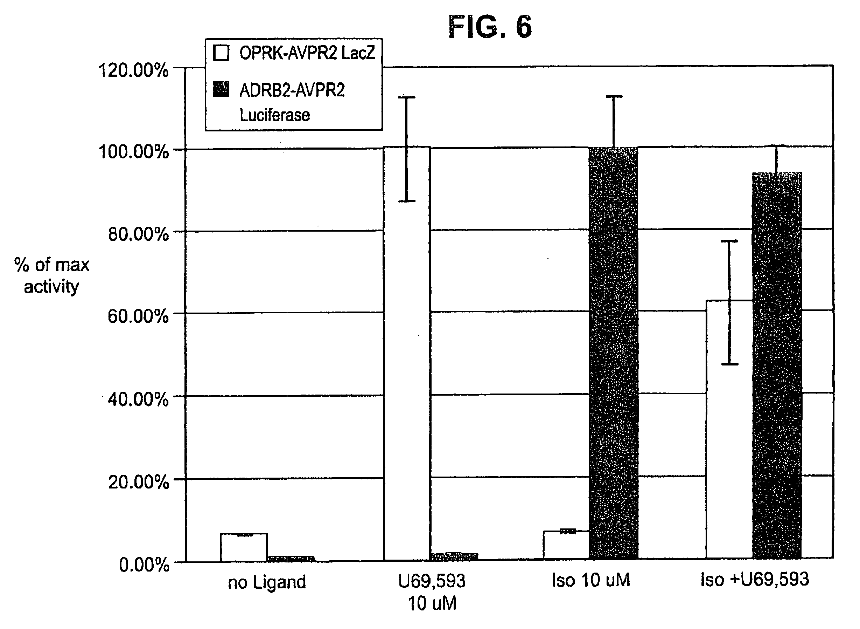

"Clone 34.9" cells, which are a derivative of clone 41 cells and containing a stably integrated ARRB2 - TEV NIa protease fusion protein gene, were transiently transfected with the chimeric OPRK-AVPR2 Tail- TEV-NIa-Pro cleavage (Leu)-tTA fusion construct described *supra.* In parallel, "clone HTL 5B8.1" cells, which are an HEK-293T cell line containing a stable integrated luciferase gene under the control of a tTA-dependent promoter, were transiently transfected with the ADRB-AVPR2 Tail- TEV-NIa-Pro cleavage (Leu)-tTA fusion construct described *supra.* In each case 5x10⁵ cells were plated in each well of a 6-well dish, and cultured for 24 hours in DMEM supplemented with 10% fetal bovine serum, 2 mM L-Glutamine, 100 units/ml penicillin, 500 µg/ml G418, and 3 µg/ml puromycin. Cells were transiently transfected with 100 µl of DMEM, 0.5 µg of OPRK-AVPR2 Tail-TEV-NIa-Pro cleavage (Leu)-tTA DNA, and 2.5 µl Fugene ("clone 34.9 cells") or with 100 µl of DMEM, 0.5 µg of ADRB2-AVPR2 Tail-TEV-NIa-Pro cleavage (Leu)-tTA DNA, 0.5 µg of ARRB2-TEV NIa Protease DNA and 5 µl Fugene ("clone HTL 5B8.1 cells"). Transiently transfected cells were cultured for about 24 hours, and were then trypsinized, mixed in equal amounts and replated in wells of a 96 well plate. Cells were incubated for 24 hours before treatment with 10 µM U-69593, 10 µM isoproterenol or a mixture of both agonists at 10 µM. Sixteen wells were assayed for each experimental condition. After 24 hours, cells were lysed and the activity of both beta-galactosidase and luciferase reporter genes were assayed as described *supra.* The results are presented in Figure 6. Treatment with U-69593 resulted in an approximately 15-fold induction of beta-galactosidase reporter gene activity, whereas luciferase activity remained unchanged. Treatment with isoproterenol resulted in a 145-fold induction of luciferase activity, while beta-galactosidase activity remained unchanged. Treatment with both U-69593 and isoproterenol resulted in nine-fold and 136-fold induction of beta-galactosidase and luciferase activity, respectively.

### COMPARATIVE EXAMPLE 19

This experiment was carried out to demonstrate the use of a different transcription factor and promoter in the assay of the invention.

A fusion construct was created, comprising DNA encoding AVPR2, fused in frame to a DNA sequence encoding the amino acid linker GSENLYFQLR (SEQ ID NO: 54) which included the low efficiency cleavage site for TEV N1a-Pro described *supra,* fused in frame to a DNA sequence encoding amino acids 2-147 of the yeast GAL4 protein (GenBank Accession Number P04386) (SEQ ID NO: 55) followed by a linker, i.e., of the sequence PELGSASAELTMVF (SEQ ID NO: 56), followed by amino acids 368-549 of the murine nuclear factor kappa-B chain p65 protein (GenBank Accession Number A37932) (SEQ ID NO: 57). The CMV promoter was placed upstream of the AVPR2 coding region and a polyA sequence was placed downstream of the GAL4-NFkB region. This construct was designated AVPR2-TEV-NIa-Pro cleavage (Leu)-GAL4.

HUL 5C1.1 is a derivative of HEK-293T cells, which contain a stably integrated luciferase reporter gene under the control of a GAL4 upstream activating sequence (UAS), commercially available pFR-LUC.

This AVPR2-TEV-NIa-Pro cleavage (Leu)-GAL4 plasmid was co-transfected along with the β-arrestin2-TEV NIa Protease described *supra* into HUL 5C1.1 cells. About 2.5 x 10⁴ cells were plated into each well of a 96 well-plate, in DMEM medium supplemented with 10% fetal bovine serum, 2 mM L-Glutamine, 100 units/ml penicillin, 500 µg/ml G418, and 3 µg/ml puromycin. Cells were grown to reach 50% confluency the next day and were transfected with 10 µl per well of a mixture consisting of 85 µl of DMEM, 0.1 µg of AVPR2-TEV-Nia-Pro cleavage (Leu)-GAL4 DNA, 0.1 µg of ARRB2-TEV NIa Protease DNA, and 1 µl Fugene, which had been incubated for 15 minutes at room temperature prior to addition to the cells. Transfected cells were cultured for about 16 hours before treatment with 10 µM vasopressin. After six hours, cells were lysed and luciferase activity was assayed as described *supra.* Under these conditions, treatment with vasopressin resulted in a 180-fold increase in reporter gene activity.

### COMPARATIVE EXAMPLE 20

This set of experiments were carried out to demonstrate enhancements of the assay using further receptor modifications that are designed to increase the affinity for the interacting protein. In this example, the C-terminal tail domain of the test receptor is replaced with the corresponding tail domain of one of the following receptors: apelin J receptor - AGTRL1 (accession number: NM_005161) (SEQ ID NO: 58), gastrin-releasing peptide receptor - GRPR (accession number: NM_005314) (SEQ ID NO: 59), proteinase-activated receptor 2 - F2RL1 (accession number: NM_005242) (SEQ ID NO: 60), CCR4 (accession number NM_005508) (SEQ ID NO: 61), chemokine (C-X-C motif) receptor 4 - CXCR4 (accession number: NM_003467) (SEQ ID NO: 62), and interleukin 8 receptor, beta - CXCR2/IL8b (accession number: NM_001557) (SEQ ID NO: 63).

First PCR was used to produce a DNA fragment encoding the C-terminal tail of the above receptors. These fragments were designed such that the first two amino acids (Ala, A and Arg, R) are encoded by the BssHII restriction site.

The AGTRL1 C-terminal fragment was amplified with the primers
tgtgcgcgcg gccagagcag gtgcgca (SEQ ID NO: 64)
and
gaggatccgt caaccacaag ggtctc (SEQ ID NO: 65).

The GRPR C-terminal fragment was amplified with the primers
tgtgcgcgcg gcctgatcat ccggtct (SEQ ID NO: 66)
and
gaggatccga cataccgctc gtgaca (SEQ ID NO: 67).

The F2RL1 C-terminal fragment was amplified with the primers
tgtgcgcgca gtgtccgcac tgtaaagc (SEQ ID NO: 68)
and
gaggatccat aggaggtctt aacagt (SEQ ID NO: 69).

The CCR4 C-terminal fragment was amplified with the primers
tgtgcgcgcg gcctttttgt gctctgc (SEQ ID NO: 70)
and
gaggatccca gagcatcatg aagatc (SEQ ID NO: 71).

The CXCR2/IL8b C-terminal fragment was amplified with the primers
tgtgcgcgcg gcttgatcag caagggac (SEQ ID NO: 72)
and
gaggatccga gagtagtgga agtgtg (SEQ ID NO: 73).

The CXCR4 C-terminal fragment was amplified with the primers
tgtgcgcgcg ggtccagcct caagatc (SEQ ID NO: 74)
and
gaggatccgc tggagtgaaa acttga (SEQ ID NO: 75).

The resulting DNA fragments encoding the modified C-terminal tail domains of these receptors were cut with BssHII and BamHI and the fragments were ligated in frame to the OPRK receptor coding region, replacing the AVPR2- C-terminal tail fragment, in the OPRK-AVPR2 Tail- TEV-NIa-Pro cleavage (Leu)-tTA expression construct described *supra.*

HTLA 5B8.1 cells described *supra* were co-transfected with each of the above modified OPRK coding region - TEV-NIa-Pro cleavage (Leu)- tTA constructs and the β-arrestin 2 - TEV NIa protease fusion described *supra.* About 2.5 x 10⁴ cells per well were plated onto a 96 well-plate, in DMEM medium supplemented with 10% fetal bovine serum, 2 mM L-Glutamine, 100 units/ml penicillin, 500 µg/ml G418, and 3 µg/ml puromycin. Cells were grown to reach 50% confluency the next day and were transfected with 10 µl per well of a mixture consisting of 85 µl of DMEM, 0.25 µg of AVPR2-TEV-NIa-Pro cleavage (Leu)-GAL4 DNA, 0.25 µg of ARRB2-TEV NIa protease DNA, and 2.5 µl Fugene (a proprietary transfection reagent containing lipids and other material), which had been incubated for 15 minutes at room temperature prior to addition to the cells. Transfected cells were cultured for about 16 hours before treatment 10 µM U-69593. After six hours, cells were lysed and luciferase activity was assayed as described *supra.* Under these conditions, treatment with U-69593 resulted in the following relative increases in reporter gene activity for each of the modified OPRK receptors: OPRK-AGTRL1 C-terminal tail - 30 fold; OPRK-GRPR C-terminal tail - 312 fold; OPRK-F2RL1 C-terminal tail - 69.5 fold; OPRK-CCR4 C-terminal tail - 3.5 fold; OPRK-CXCR4 C-terminal tail - 9.3 fold; OPRK-IL8b C-terminal tail - 113 fold.

### COMPARATIVE EXAMPLE 21

This experiment was designed to produce a cell line that stably expressed the ARRB2-TEV NIa protease fusion protein described *supra.*

A plasmid was made which expressed the ARRB2-TEV NIa protease fusion protein under the control of the EF1α promoter and also expressed the hygromycin resistance gene under the control of the thymidine kinase (TK) promoter.

This plasmid was transfected into HTL 5B8.1, and clones containing a stable genomic integration of the plasmid were selected by culturing in the presence of 100 µg/ml hygromycin. Resistant clones were isolated and expanded and were screened by transfection of the ADRB2-AVPR2 Tail- TEV-NIa-Pro cleavage (Leu)-tTA plasmid described *supra.* Three cell lines that were selected using this procedure were designated "HTLA 4C2.10", "HTLA 2C11.6" and "HTLA 5D4". About 2.5 x 10⁴ cells per well were plated onto a 96 well-plate, in DMEM medium supplemented with 10% fetal bovine serum, 2 mM L-Glutamine, 100 units/ml penicillin, 500 µg/ml G418, 3 µg/ml puromycin, and 100 µg/ml hygromycin. Cells were grown to reach 50% confluency the next day and were transfected with 10 µl per well of a mixture consisting of 85 µl of DMEM, 0.25 µg of ADRB2-AVPR2-TEV-NIa-Pro cleavage (Leu)-GAL4 DNA and 0.5 µl Fugene, which had been incubated for 15 minutes at room temperature prior to addition to the cells. Transfected cells were cultured for about 16 hours before treatment 10 µM isoproterenol. After six hours, cells were lysed and luciferase activity was assayed as described *supra.* Under these conditions, treatment with isoproterenol resulted in a 112-fold ("HTLA 4C2.10"), 56-fold ("HTLA 2C11.6") and 180-fold ("HTLA 5D4") increase in reporter gene activity in the three cell lines, respectively.

### COMPARATIVE EXAMPLE 22

This experiment was designed to produce a cell line that stably expressed the ARRB2-TEV NIa protease and the ADRB2-AVPR2 Tail- TEV-NIa-Pro cleavage (Leu)-tTA fusion proteins described *supra.*

The ARRB2-TEV NIa protease plasmid containing the hygromycin resistance gene was transfected together with the ADRB2-AVPR2 Tail- TEV-NIa-Pro cleavage (Leu)-tTA fusion protein plasmid described *supra* into HTL 5B8.1 cells and clones containing stable genomic integration of the plasmids were selected by culturing in the presence of 100 µg/ml hygromycin. Resistant clones were isolated and expanded, and were screened by treating with 10 µM isoproterenol and measuring the induction of reporter gene activity as described *supra.* Three cell lines that were selected using this procedure were designated "HTLAR 1E4", "HTLAR 1C10" and "HTLAR 2G2". Treatment with isoproterenol for 6 hours resulted in a 208-fold ("HTLAR 1E4"), 197-fold ("HTLAR 1C10") and 390-fold ("HTLAR 2G2') increase in reporter gene activity in the three cell lines, respectively.

### COMPARATIVE EXAMPLE 23

This experiment was designed to demonstrate the use of the assay to measure the activity of the receptor tyrosine kinase epidermal growth factor receptor (EGFR).

A first fusion construct was created, comprising DNA encoding the human EGFR, which can be found at GenBank under the Accession Number MM_005228 (SEQ ID NO: 76), fused in frame to a DNA sequence encoding amino acids 3-335 of the tetracycline-controlled transactivator tTA, described *supra.* Inserted between these sequences is a DNA sequence encoding the amino acid sequence GGSGSENLYFQL (SEQ ID NO: 77) which includes the low efficiency cleavage site for TEV NIa-Pro, ENLYFQL (SEQ ID NO: 14), described *supra.* The CMV promoter was placed upstream of the Epidermal Growth Factor Receptor coding region, and a polyA sequence was placed downstream of the tTA region. This construct is designated EGFR-TEV-NIa-Pro cleavage (Leu)-tTA.

A second fusion construct was created, comprising DNA encoding the two SH2 domains of human Phospholipase C Gamma 1, corresponding to amino acids 538-759 (GencBank accession number NP_002651.2) (SEQ ID NO: 78) fused in frame to a DNA sequence encoding the catalytic domain of mature TEV NIa protease, described *supra,* corresponding to amino acids 2040-2279 (GeneBank accession number AAA47910) (SEQ ID NO: 79). Inserted between these sequences is a linker DNA sequence encoding the amino acids NSSGGNSGS (SEQ ID NO: 80). The CMV promoter was placed upstream of the PLC-Gamma SH2 domain coding sequence and a polyA sequence was placed downstream of the TEV NIa protease sequence. This construct is designated PLC Gamma1-TEV.

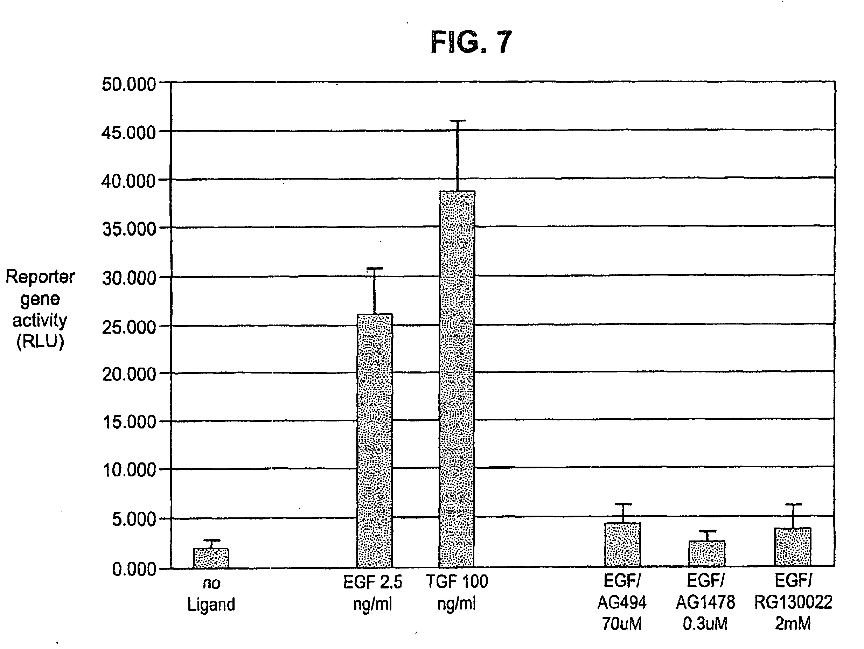

The EGFR-TEV-NIa-Pro cleavage (Leu)-tTA and PLC Gamma1-TEV fusion constructs were transfected into clone HTL5B8.1 cells described *supra.* About 2.5 x 10⁴ cells were plated into each well of a 96 well-plate, in DMEM medium supplemented with 10% fetal bovine serum, 2 mM L-Glutamine, 100 units/ml penicillin, 500 µg/ml G418, and 3 µg/ml puromycin. Cells were grown to reach 50% confluency the next day and were transfected with 15 µl per well of a mixture consisting of 100 µl of DMEM, 0.4 µg of pcDNA3 DNA ("carrier" vector DNA), 0.04 µg of EGFR-TEV-NIa-Pro cleavage (Leu)-tTA DNA, 0.04 µg of PLC Gamma1-TEV DNA, and 2 µl Fugene (a proprietary transfection reagent containing lipids and other material), which had been incubated for 15 minutes at room temperature prior to addition to the cells. Transfected cells were cultured for about 16 hours before treatment with specified receptor agonists and inhibitors. After six hours, cells were lysed and luciferase activity was assayed as described *supra.* Results are shown in Figure 7.

The addition of 2.5 ng/ml human Epidermal Growth Factor (corresponding to the EC80 for this ligand) resulted in a 12.3 fold increase of luciferase reporter gene activity, while addition of 100 ng/ml human Transforming Growth Factor - Alpha resulted in an 18.3 fold increase. Prior treatment with tyrosine kinase inhibitors (70 µM AG-494; 0.3 µM AG-1478; 2 mM RG-130022) before addition of human Epidermal Growth Factor blocked the induction of reporter gene activity.

### COMPARATIVE EXAMPLE 24

This experiment was designed to demonstrate the use of the assay to measure the activity of the human Type I Interferon Receptor.

A fusion construct was created, comprising DNA encoding human Interferon Receptor I (IFNAR1) (557 amino acids), which can be found in Genbank under Accession Number NM_000629 (SEQ ID NO: 81), fused in frame to a DNA sequence encoding amino acids 3-335 of the tetracycline controlled transactivator tTA, described *supra.* Inserted between these sequences is a DNA sequence encoding the amino acid sequence GSENLYFQL (SEQ ID NO: 82) which includes the low efficiency cleavage site for TEV NIa-Pro, ENLYFQL (SEQ ID NO: 14), described *supra.* The CMV promoter was placed upstream of the Human Interferon Receptor I (IFNAR1) coding region, and a poly A sequence was placed downstream of the tTA region. This construct is designated IFNAR1- TEV-NIa-Pro cleavage (L)-tTA.

A second fusion construct was created, using DNA encoding Human Interferon Receptor 2, splice variant 2 (IFNAR2.2) (515 amino acids), which can be found at Genbank, under Accession Number L41942 (SEQ ID NO: 83), fused in frame to a DNA sequence encoding the catalytic domain of the TEV NIa protease, described *supra,* corresponding to amino acids 2040-2279 (GenBank accession number AAA47910) (SEQ ID NO: 84). Inserted between these sequences is a DNA sequence encoding the amino acid sequence RS (Arg-Ser). The CMV promoter region was placed upstream of the Human Interferon Receptor 2 (IFNAR2.2) coding region, and a poly A sequence was placed downstream of the TEV region. This construct is designated IFNAR2.2-TEV.

Expression constructs were also generated in which the genes for Human Signal Transducer and Activator of Transcription 1 (STAT1), found in Genbank, under Accession Number NM_007315 (SEQ ID NO: 85), Human Signal Transducer and Activator of Transcription 2 (STAT2) found in Genbank, under Accession Number NM_005419 (SEQ ID NO: 86), were expressed under the control of the CMV promoter region. These constructs were designated CMV-STAT1 and CMV-STAT2 respectively.

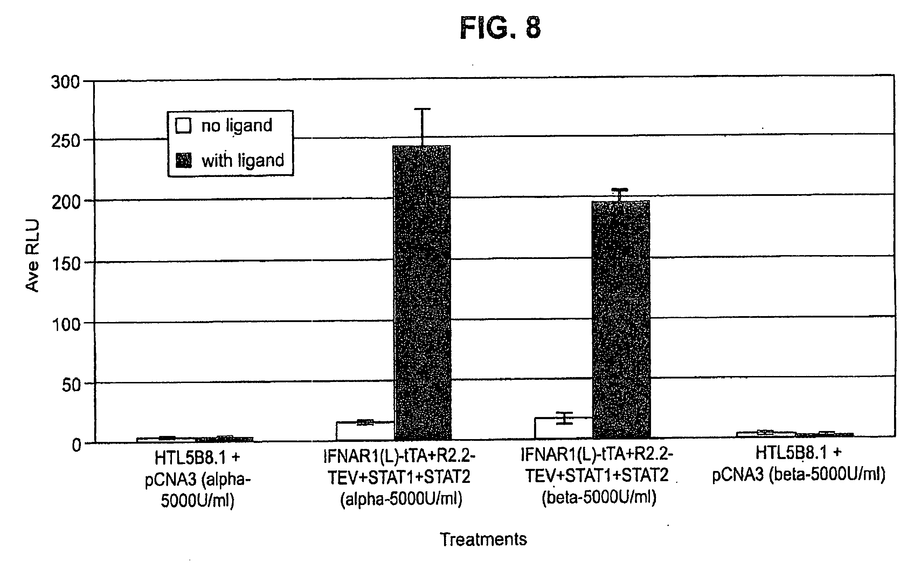

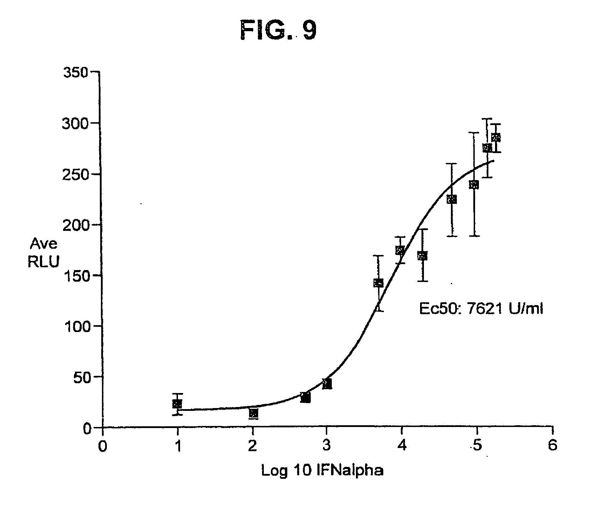

The IFNAR1- TEV- NIa-Pro cleavage (L)-tTA and IFNAR2.2-TEV fusion constructs, together with CMV-STAT1 and CMV-STAT2 were transiently transfected into HTLSB8.1 cells described *supra.* About 2.5 x 10⁴ cells were seeded in each well of a 96 well plate and cultured in DMEM medium supplemented with 10% fetal bovine serum, 2mM L-glutamine, 100 units/ml penicillin, 100µg/ml G418, and 5 µg/ml puromycin. After 24 hours of incubation, cells were transfected with 15ng of each IFNAR1- TEV- NIa-Pro cleavage (L)-tTA, IFNAR2.2-TEV, CMV-STAT1 and CMV-STAT2 DNA, or with 60ng control pcDNA plasmid, together with 0.3 µl Fugene per well. Transfected cells were cultured for 8-20 hours before treatment with 5000 U/ml human interferon-alpha or 5000 U/ml human interferon-beta. At the time of interferon addition, medium was aspirated and replaced with 293 SFM II media supplemented with 2mM L-glutamine, 100 units/ml penicillin, 3µg/ml puromycin and 500µg/ml of G418. Interferon-treated cells were cultured for an additional 18-20 hours before they were assayed for luciferase reporter gene activity as described *supra.* Results are shown in Figure 8. Treatment with 5000U/ml IFN-α resulted in 15-fold increase in reporter gene activity, while treatment with 5000U/ml IFN-β resulted in a 10-fold increase. Interferon treatment of HTL5B8.1 cells transfected with the control plasmid pcDNA3 had no effect on reporter gene activity. Figure 9 shows a dose-response curve generated for IFN-α in HTL5B8.1 cells transfected with IFNAR1 (ENLYFQ(L)-tTa, IFNAR2.2-TEV, STAT1 and STAT2 expression constructs as described *supra.*

### COMPARATIVE EXAMPLE 25

This experiment was designed to demonstrate the use of the assay to measure the activity of the human Type I Interferon Receptor using a different transcription factor and a different cell line.

A fusion construct was created, using DNA encoding Human Interferon Receptor I (IFNAR1), fused in frame to a DNA sequence encoding the GAL4-NF-κB-fusion, described *supra.* Inserted between these sequences is a DNA sequence encoding the amino acid sequence GSENLYFQL (SEQ ID NO: 87), which includes the low efficiency cleavage site for TEV NIa-Pro, ENLYFQL (SEQ ID NO: 14), described *supra.* The CMV promoter was placed upstream of the Human Interferon Receptor I (IFNAR1) coding region, and a poly A sequence was placed downstream of the GAL4-NF-κB region. This construct is designated IFNAR1-TEV- NIa-Pro cleavage (L)-GAL4-NF-κB.

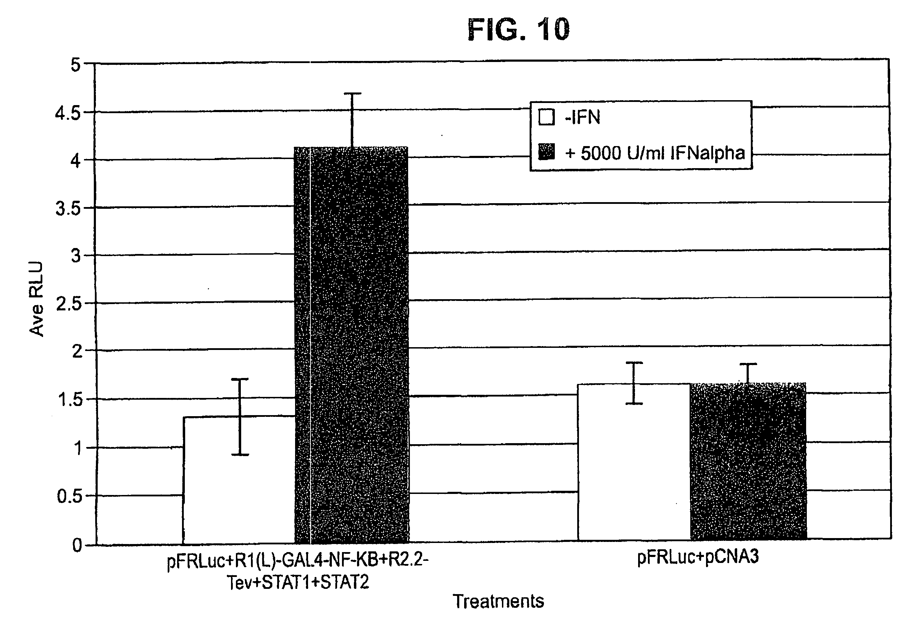

CHO-K1 cells were then transiently transfected with a mixture of five plasmids: IFNAR1-TEV-NIa-Pro cleavage (L)-GAL4-NF-κB, IFNAR2.2-TEV, CMV-STAT1, CMV-STAT2 and pFR-Luc, a luciferase reporter gene plasmid under the control of a GAL4-dependent promoter. About 1.0 x 10⁴ cells per well were seeded in a 96 well plate 24 hours prior to transfections in DMEM medium supplemented with 10% fetal bovine serum, 2mM L-glutamine, 100 units/ml penicillin. Cells were transfected the following day with 10 ng of reporter plasmid (pFR-Luc), plus 20 ng of each of the expression constructs described *supra,* or with 10 ng reporter plasmid plus 80 ng of control pcDNA3 plasmid, together with 0.3 µl Fugene per well. Transfected cells were cultured for 8-20 hours before treatment with 5000 U/ml human interferon-alpha. At the time of interferon addition, medium was aspirated and replaced with DMEM media supplemented with 2mM L-glutamine, 100 units/ml penicillin. Interferon-treated cells were cultured for an additional 6 hours before they were assayed for luciferase reporter gene activity as described *supra.* Results are shown in Figure 10. IFN-α treatment of CHO-K1 cells transfected with the reported, IFNAR and STAT constructs resulted in 3-fold increase in reporter gene activity, while interferon treatment of cells transfected with the reporter and control plasmids had no effect on reporter gene activity.

### COMPARATIVE EXAMPLE 26

This set of experiments was carried out to demonstrate additional enhancements of the assay using receptor modifications designed to increase the affinity of the test receptor for the interacting protein. In these examples, the fusion junction between the test receptor and a C-terminal tail domain of GRPR (Genbank Accession Number: NM_005314) (SEQ ID NO: 59) was made 17-23 amino acids after the conserved NPXXY motif at the end of the seventh transmembrane helix.

First, PCR was used to produce a DNA fragment encoding the C-terminal 42 amino acids from GRPR beginning 2 amino acids after the putative palmitoylation site (hereafter referred to as GRPR 42aa). The fragment was designed such that the first amino acid of the C-terminal tail is preceded by two amino acids (Ser, S and Arg, R) which are encoded by the XbaI restriction site TCTAGA, and the stop codon is replaced by two amino acids (Gly, G and Ser, S) which are encoded by a BamHI restriction site GGATCC. This was accomplished by amplifying a plasmid containing the GRPR coding region with primers
tctagaggcctgatcatccggtctcac (SEQ ID NO: 88)
and
gaggatccgacataccgctcgtgaca (SEQ ID NO: 67)

Next the coding region of OPRK (Genbank Accession Number: NM_000912) (SEQ ID NO: 51) was modified to place insert an XbaI site after Pro-347. This was done using PCR with the primers
ggtctacttgatgaattcctggcc (SEQ ID NO: 52)
and
tctagatggaaaacagaagtcccggaaac (SEQ ID NO: 89)

In addition, the coding region of ADRA1A (Genbank Accession Number: NM_000680) (SEQ ID NO: 90) was modified to insert an XbaI site after Lys-349. This was done using PCR with the primers
ctcggatatctaaacagctgcatcaa (SEQ ID NO: 91)
and
tctagactttctgcagagacactggattc (SEQ ID NO: 92)

In addition, the coding region of DRD2 (Genbank Accession Number: NM_000795) (SEQ ID NO: 37) was modified to insert two amino acids (Leu and Arg) and an XbaI site after Cys-343. This was done using PCR with the primers
gaattcatggatccactgaatctgtcc (SEQ ID NO: 38)
and
tctagatcgaaggcagtggaggatcttcagg (SEQ ID NO: 93)

The modified OPRK receptor coding region was cut with EcoRI and XbaI and the GRPR 42aa C-terminal tail fragment was cut with XbaI and BamHI. Both fragments were ligated into a plasmid containing the OPRK receptor with the AVPR2 C-terminal tail-low-efficiency cleavage site-tTA described *supra* which had been digested with EcoRI and BamHI.

The modified ADRA1A receptor coding region was cut with EcoRV and XbaI and the OPRK-GRPR 42aa Tail-tTA fusion construct containing the low efficiency cleavage site was cut with XbaI and XhoI. Both fragments were ligated into a plasmid containing the ADRA1A receptor which had been digested with EcoRV and XhoI.

The modified DRD2 receptor coding region was cut with EcoRI and XbaI and the OPRK-GRPR 42aa Tail-tTA fusion construct containing the low efficiency cleavage site was cut with XbaI and XhoI. Both fragments were ligated into a pcDNA6 plasmid digested with EcoRI and XhoI

HTLA 2C11.6 cells, described *supra,* were transfected with OPRK-GRPR 42aa Tail-tTA fusion construct containing the low efficiency cleavage site and assays were carried out using 10 µM U-69593, an agonist for OPRK. The maximal response to U-69593 resulted in an approximately 200-fold increase in reporter gene activity.

HTLA 2C11.6 cells were transfected with ADRA1A-GRPR 42aa Tail-tTA fusion construct containing the low efficiency cleavage site and assays were carried out using 10 µM epinephrine, an agonist for ADRA1A. The maximal response to epinephrine resulted in an approximately 14-fold increase in reporter gene activity.

HTLA 2C11.6 cells were transfected with DRD2-GRPR 42aa Tail-tTA fusion construct containing the low efficiency cleavage site and assays were carried out using 10 µM dopamine, an agonist for DRD2. The maximal response to dopamine resulted in an approximately 30-fold increase in reporter gene activity.

### COMPARATIVE EXAMPLE 27

This set of experiments were carried out to demonstrate further enhancements of the assay using a different set of test receptor modifications designed to increase the affinity for the interacting protein. In these examples, the C-terminal domain of the test receptor was replaced with a portion of the endogenous C-terminal tail domain of GRPR.

First, PCR was used to produce a DNA fragment encoding the truncated GRPR tail, specifically a sequence encoding 23 amino acids from Gly-343 to Asn-365. The fragment was designed such that the first amino acid of the C-terminal tail is preceded by two amino acids (Ser, S and Arg, R) which are encoded by the XbaI restriction site TCTAGA, and the Ser-366 is replaced by two amino acids (Gly, G and Ser, S) which arc encoded by a BamHI restriction site GGATCC. This was accomplished by amplifying a plasmid containing the GRPR coding region with primers
tctagaggcctgatcatccggtctcac (SEQ ID NO: 94)
and
cggatccgttggtactcttgagg (SEQ ID NO: 95)

Next the truncated GRPR fragment (hereafter referred to as GRPR 23aa Tail) was cut with XbaI and BamHI and inserted into the OPRK-GRPR 42aa Tail-tTA fusion construct containing the low efficiency cleavage site described herein, digested with XbaI and BamHI.

Similarly, the GRPR 23aa Tail fragment was cut with XbaI and BamHI and inserted into the ADRA1A-GRPR 42aa Tail-tTA fusion construct containing the low efficiency cleavage site described herein, digested with XbaI and BamHI.

HTLA 2C11.6 cells were transfected with OPRK-GRPR 23aa Tail -tTA fusion construct containing the low efficiency cleavage site and assays were carried out using 10 µM U-69593, an agonist for OPRK. The maximal response to U-69593 resulted in an approximately 115-fold induction of reporter gene expression over the background

HTLA 2C11.6 cells were transfected with ADRA1A-GRPR 23aa Tail-tTA fusion construct containing the low efficiency cleavage site and assays were carried out using 10 µM epinephrine, an agonist for ADRA1A. The maximal response to epinephrine resulted in an approximately 102-fold induction of reporter gene expression over the background.

### COMPARATIVE EXAMPLE 28

This experiment was designed to demonstrate the use of the assay to measure the activity of the receptor tyrosine kinase Insulin-like Growth Factor-1 Receptor (IGF1R), specifically by monitoring the ligand-induced recruitment of the intracellular signaling protein SHC1 (Src homology 2 domain-containing transforming protein 1).

A first fusion construct was created, comprising DNA encoding the human IGF-1R, which can be found at GenBank under the Accession Number NM_000875 (SEQ ID NO: 96), fused in frame to a DNA sequence encoding amino acids 3-335 of the tetracycline-controlled transactivator tTA, described *supra.* Inserted between these sequences is a DNA sequence encoding the amino acid sequence GSENLYFQL (SEQ ID NO: 82) which includes the low efficiency cleavage site for TEV NIa-Pro, ENLYFQL (SEQ ID NO: 14), described *supra.* The CMV promoter was placed upstream of the IGFIR coding region, and a polyA sequence was placed downstream of the tTA region. This construct is designated IGF1R-TEV-NIa-Pro cleavage (Leu)-tTA.

A second fusion construct was created, comprising DNA encoding the PTB domain of human SHC1, corresponding to amino acids 1-238 (GeneBank accession number BC014158) (SEQ ID NO: 97) fused in frame to a DNA sequence encoding the catalytic domain of mature TEV NIa protease, described *supra,* corresponding to amino acids 2040-2279 (GeneBank accession number AAA47910) (SEQ ID NO: 79). Inserted between these sequences is a linker DNA sequence encoding the amino acids NSGS (SEQ ID NO: 98). The CMV promoter was placed upstream of the SHC1 PTB domain coding sequence and a polyA sequence was placed downstream of the TEV NIa protease sequence. This construct is designated SHC1-TEV.

The IGF1R-TEV-NIa-Pro cleavage (Leu)-tTA and SHC1-TEV fusion constructs were transfected into clone HTLSB8.1 cells described *supra.* About 2.5 x 10⁴ cells were plated into each well of a 96 well-plate, in DMEM medium supplemented with 10% fetal bovine serum, 2 mM L-Glutamine, 100 units/ml penicillin, 500 µg/ml G418, and 3 µg/ml puromycin. Cells were grown to reach 50% confluency the next day and were transfected with 15 µl per well of a mixture consisting of 100 µl of DMEM, 0.2 µg of IGFIR-TEV-NIa-Pro cleavage (Leu)-tTA DNA, 0.2 µg of SHCl-TEV DNA, and 2 µl Fugene (a proprietary transfection reagent containing lipids and other material), which had been incubated for 15 minutes at room temperature prior to addition to the cells. Transfected cells were cultured for about 16 hours before treatment with a specific receptor agonist. After 24 hours, cells were lysed and luciferase activity was assayed as described *supra.*

The addition of 1 µM human Insulin-like Growth Factor 1 resulted in a 90 fold increase of luciferase reporter gene activity.

### EXAMPLE 29

This experiment was designed to demonstrate the use of the assay to measure the interaction of two test proteins that are not normally membrane bound. In this example, the assay was used to measure the ligand-induced dimerization of the nuclear steroid hormone receptors, ESR1 (estrogen receptor I or ER alpha) and ESR2 (estrogen receptor 2 or ER beta). In this example, ESR1 is fused to the transcription factor tTA, where the cleavage site for the TEV NIa-Pro protease is inserted between the ESR1 and tTA sequences. This ESR1-tTA fusion is tethered to the membrane by a fusion to the intracellular, C-terminal end of the transmembrane protein CD8. CD8 essentially serves as an inert scaffold that tethers ESR1 to the cytoplasmic side of the cell membrane. The transcription factor fused thereto cannot enter the nucleus until interaction with ESR2 and protease. Any transmembrane protein could be used. This CD8-ESR1-TEV NIa Pro cleavage-tTA fusion protein is expressed together with a second fusion protein comprised of ESR2 and the TEV NIa-Pro protease in a cell line containing a tTA-dependent reporter gene. The estrogen-induced dimerization of ESR1 and ESR2 thereby triggers the release of the tTA transcription factor from the membrane bound fusion, which is detected by the subsequent induction in reporter gene activity.

A fusion construct was created, comprising DNA encoding human CD8 gene (235 amino acids), which can be found in Genbank under Accession Number NM_001768 (SEQ ID NO: 99), fused in frame to a DNA sequence encoding the human ESR1 (596 amino acids), which can be found in Genbank under Accession Number NM_000125 (SEQ ID NO: 100). Inserted between these sequences is a DNA sequence encoding the amino acid sequence GRA (Gly-Arg-Ala). The resulting construct is then fused in frame to a DNA sequence encoding amino acids 3-335 of the tetracycline controlled transactivator tTA, described *supra.* Inserted between these sequences is a DNA sequence encoding the amino acid sequence GSENLYFQL (SEQ ID NO: 82) which includes the low efficiency cleavage site for TEV NIa-Pro, ENLYFQL (SEQ ID NO: 14), described *supra.* The CMV promoter was placed upstream of the Human CD8 coding region, and a poly A sequence was placed downstream of the tTA region. This construct is designated CD8-ESR1- TEV- NIa-Pro cleavage (L)-tTA.

A second fusion construct was created, using DNA encoding Human Estrogen Receptor beta (ESR2) (530 amino acids), which can be found at Genbank, under Accession Number NM_001437 (SEQ ID NO: 101), fused in frame to a DNA sequence encoding the catalytic domain of the TEV NIa protease, described *supra,* corresponding to amino acids 2040-2279 (GenBank accession number AAA47910) (SEQ ID NO: 84). Inserted between these sequences is a DNA sequence encoding the amino acid sequence RS (Arg-Ser). The CMV promoter region was placed upstream of the Human Estrogen Receptor beta (ESR2) coding region, and a poly A sequence was placed downstream of the TEV region. This construct is designated ESR2-TEV.

The CD8-ESR1- TEV-NIa-Pro cleavage (L)-tTA and ESR2-TEV fusion constructs, together with pCDNA3 were transiently transfected into HTL5B8.1 cells described *supra.* About 2.0 x 10⁴ cells were seeded in each well of a 96 well plate and cultured in phenol-free DMEM medium supplemented with 10% fetal bovine serum, 2mM L-glutamine, 100 units/ml penicillin, 100 µg/ml G418, and 5 µg/ml puromycin. After 24 hours of incubation, cells were transfected with a mixture of 5ng of ESR1- TEV- NIa-Pro cleavage (L)-tTA, 15ng of ESR2-TEV and 40ng of pCDNA3, together with 0.3 µl Fugene per well. 6 hours after transfection, the cells were washed with PBS and incubated in 100µl of phenol-free DMEM without serum for 24 hours before treatment with 50nM 17-β Estradiol. Ligand-treated cells were cultured for an additional 18-20 hours before they were assayed for luciferase reporter gene activity as described *supra.* Treatment with 50mM 17-β Estradiol resulted in a 16-fold increase in reporter gene activity.

Other features of the invention will be clear to the skilled artisan and need not be reiterated here.

## Claims

1. A method for determining if a test compound modulates a specific protein/protein interaction of interest, comprising contacting said compound to a cell which has been transfected or transformed with
(a) a first nucleic acid molecule which encodes a first fusion protein, the first nucleic acid molecule comprising:
(i) a nucleotide sequence which encodes said first test protein,
(ii) a nucleotide sequence encoding a specific cleavage site for a protease or a portion of a protease not endogenously expressed by said cell, and
(iii) a nucleotide sequence which encodes either (Y) a first, inactive portion of a protein, which completes a second, inactive portion of said protein in said cell, to produce a complete, functioning protein which produces a direct or indirect signal, or (Z) a protein or portion of a protein which upon release from the fusion protein can be tracked as it moves, redistributes, translocates or otherwise changes position within the cell;
(b) a second nucleic acid molecule which encodes a second fusion protein, the second nucleic acid molecule comprising
(iv) a nucleotide sequence which, encodes a second test protein whose interaction with said first protein in the presence of said test compound is to be measured, and
(v) a nucleotide sequence which encodes the protease or a portion of the protease which is specific for said cleavage site in the first fusion protein;
and determining a signal resulting from release of (a)(iii) from (a)(i) and (a)(ii) as a determination of modulation of said protein/protein interaction by said compound.

2. The method of claims 1, comprising determining localization of said protein or portion of protein (a)(iii) to an organelle.

3. The method of claim 1, wherein said protein or protein portion of (a)(iii) is a fluorescent protein or a portion of a fluorescent protein.

4. The method of claim 3, wherein said fluorescent protein or portion of a fluorescent protein is green fluorescent protein, red fluorescent protein, cyan fluorescent protein, a yellow fluorescent protein, or a portion thereof.

5. The method of claim 1, wherein said first and second inactive portions of a protein (Y) are portions of an enzyme.

6. The method of claim 5, wherein said enzyme is an enzyme which catalyzes a reaction leading to a detectable signal.

7. The method of claim 6, wherein said enzyme is nitroreductase, luciferase, beta galactosidase, aequorin, beta glucuronidase, beta lactamase, or alkaline phosphatase.

8. The method of claim 1, wherein (Z) is a protein or protein portion which binds specifically to an antibody or binding antibody fragment located within said cell, or an epitopic portion of said protein.

9. The method of claim 1, wherein (Z) is an antibody or binding antibody fragment which binds specifically to an epitopic protein located within said cell.

10. A recombinant cell, transformed or transfected with nucleic acid molecules (a) and (b) as defined by any of claims 1 and 3 to 9.

11. The cell of claim 10 wherein one or both of the nucleic acid molecules are stably incorporated into the genome of the cell.

12. The cell of claim 10 or claim 11 wherein the first test protein is a membrane bound protein, such as a transmembrane receptor, a GPCR, a cellular receptor, an ion channel, a growth factor receptor, or a cytokine receptor.

13. A test kit useful for determining if a test compound modulates a specific protein/protein interaction of interest, comprising a separate portion of each of the nucleic acid molecule constructs (a) and (b) as defined by any of claims 1 and 3 to 9, optionally wherein the first test protein is a membrane bound protein, such as a transmembrane receptor, and/or the second protein is an inhibitory protein, such as an arrestin.

## Patentansprüche

1. Verfahren zur Bestimmung, ob eine Testverbindung eine spezifische Protein-Protein-Wechselwirkung von Interesse moduliert, wobei das Verfahren das Inkontaktbringen der Verbindung mit einer Zelle, die mit folgendem transfiziert oder transformiert wurde:
(a) einem ersten Nukleinsäuremolekül, das ein erstes Fusionsprotein codiert, wobei das erste Nukleinsäuremolekül Folgendes umfasst:
(i) eine Nukleotidsequenz, die das erste Testprotein codiert,
(ii) eine Nukleotidsequenz, die eine spezifische Spaltstelle für eine Protease oder einen Teil einer Protease codiert, die bzw. der von der Zelle nicht endogen exprimiert wird, und
(iii) eine Nukleotidsequenz, die entweder (Y) einen ersten inaktiven Teil eines Proteins, der einen zweiten inaktiven Teil des Proteins in der Zelle ergänzt, um ein vollständiges, funktionierendes Protein zu produzieren, das ein direktes oder indirektes Signal produziert, oder (Z) ein Protein oder einen Teil eines Proteins, das bzw. der bei Freisetzung von dem Fusionsprotein verfolgt werden, während es bzw. er sich bewegt, neu verteilt, transloziert oder anderweitig seine Position in der Zelle verändert, codiert;
(b) einem zweiten Nukleinsäuremolekül, das ein zweites Fusionsprotein codiert, wobei das zweite Nukleinsäuremolekül Folgendes umfasst:
(iv) eine Nukleotidsequenz, die ein zweites Testprotein codiert, dessen Wechselwirkung mit dem ersten Protein in Gegenwart der Testverbindung gemessen werden soll, und
(v) eine Nukleotidsequenz, die die Protease oder einen Teil der Protease codiert, die bzw. der für die Spaltstelle in dem ersten Fusionsprotein spezifisch ist;
und das Bestimmen eines Signals, das aus der Freisetzung von (a)(iii) aus (a) (i) und (a)(ii) resultiert, als eine Bestimmung der Modulierung der Protein-Protein-Wechselwirkung durch die Verbindung umfasst.

2. Verfahren nach Anspruch 1, das die Bestimmung der Lokalisierung des Proteins oder des Teils des Proteins (a)(iii) zu einer Organelle umfasst.

3. Verfahren nach Anspruch 1, wobei das Protein oder der Proteinteil von (a)(iii) ein fluoreszierendes Protein oder ein Teil eines fluoreszierenden Proteins ist.

4. Verfahren nach Anspruch 3, wobei das fluoreszierende Protein oder der Teil eines fluoreszierenden Proteins grün fluoreszierendes Protein, rot fluoreszierendes Protein, cyan fluoreszierendes Protein, ein gelb fluoreszierendes Protein oder ein Teil davon ist.

5. Verfahren nach Anspruch 1, wobei der erste und der zweite inaktive Teil eines Proteins (A) Teile eines Enzyms sind.

6. Verfahren nach Anspruch 5, wobei das Enzym ein Enzym ist, das eine Reaktion katalysiert, die zu einem nachweisbaren Signal führt.

7. Verfahren nach Anspruch 6, wobei das Enzym Nitroreduktase, Luciferase, Beta-Galaktosidase, Äquorin, Beta-Glukuronidase, Beta-Laktamase oder alkalische Phosphatase ist.

8. Verfahren nach Anspruch 1, wobei (Z) ein Protein oder Proteinteil, das bzw. der spezifisch an einen Antikörper oder ein bindendes Antikörperfragment, der bzw. das sich in der Zelle befindet, bindet, oder ein epitoper Teil des Proteins ist.

9. Verfahren nach Anspruch 1, wobei (Z) ein Antikörper oder bindendes Antikörperfragment ist, der bzw. das spezifisch an ein epitopes Protein bindet, das sich in der Zelle befindet.

10. Rekombinante Zelle, die mit den Nukleinsäuremolekülen (a) und (b) wie in einem der Ansprüche 1 und 3 bis 9 definiert transformiert oder transfiziert wurde.

11. Zelle nach Anspruch 10, wobei eines oder beide der Nukleinsäuremoleküle stabil in das Genom der Zelle integriert ist bzw. sind.

12. Zelle nach Anspruch 10 oder 11, wobei das erste Testprotein ein membrangebundenes Protein ist, wie ein Transmembranrezeptor, ein GPCR, ein zellulärer Rezeptor, ein Ionenkanal, ein Wachstumsfaktorrezeptor oder ein Zytokinrezeptor.

13. Testkit, das zur Bestimmung, ob eine Testverbindung eine spezifische Protein-Protein-Wechselwirkung von Interesse moduliert, von Nutzen ist, wobei das Testkit einen separaten Teil jedes der Nukleinsäuremolekülkonstrukte (a) und (b) wie in einem der Ansprüche 1 und 3 bis 9 definiert umfasst, gegebenenfalls wobei das erste Testprotein ein membrangebundenes Protein ist, wie ein Transmembranrezeptor, und/oder das zweite Protein ein inhibitorisches Protein ist, wie ein Arrestin.

## Revendications

1. Procédé pour déterminer si un composé d'essai module une interaction spécifique protéine/protéine d'intérêt, comprenant la mise en contact dudit composé avec une cellule qui a été transfectée ou transformée par
(a) une première molécule d'acide nucléique qui code pour une première protéine de fusion, la première molécule d'acide nucléique comprenant :
(i) une séquence nucléotidique qui code pour ladite première protéine d'essai ;
(ii) une séquence nucléotidique codant pour un site de clivage spécifique pour une protéase ou une partie d'une protéase qui n'est pas exprimée de manière endogène par ladite cellule ; et
(iii) une séquence nucléotidique qui code soit pour (Y) une première partie, inactive, d'une protéine, qui complète une seconde partie, inactive, de ladite protéine dans ladite cellule, pour produire une protéine opérationnelle complète qui produit un signal direct ou indirect, soit pour (Z) une protéine ou partie d'une protéine qui, lors de la libération à partir de la protéine de fusion, peut être tracée alors qu'elle est en mouvement, redistribuée, transloquée ou change autrement de position à l'intérieur de la cellule ;
(b) une seconde molécule d'acide nucléique qui code pour une seconde protéine de fusion, la seconde molécule d'acide nucléique comprenant
(iv) une séquence nucléotidique qui code pour une seconde protéine d'essai dont l'interaction avec ladite première protéine en présence dudit composé d'essai doit être mesurée ; et
(v) une séquence nucléotidique qui code pour la protéase ou une partie de la protéase qui est spécifique pour ledit site de clivage dans la première protéine de fusion ;
et la détermination d'un signal résultant de la libération de (a) (iii) à partir de (a)(i) et (a)(ii) en tant que détermination de modulation de ladite interaction protéine/protéine par ledit composé.

2. Procédé selon la revendication 1, comprenant la détermination de la localisation de ladite protéine ou partie de protéine (a)(iii) dans une organelle.

3. Procédé selon la revendication 1, dans lequel ladite protéine ou partie de protéine de (a)(iii) est une protéine fluorescente ou une partie d'une protéine fluorescente.

4. Procédé selon la revendication 3, dans lequel ladite protéine fluorescente ou partie d'une protéine fluorescente est une protéine fluorescente verte, une protéine fluorescente rouge, une protéine fluorescente cyan, une protéine fluorescente jaune ou une partie de celles-ci.

5. Procédé selon la revendication 1, dans lequel lesdites première et seconde parties inactives d'une protéine (Y) sont des parties d'une enzyme.

6. Procédé selon la revendication 5, dans lequel ladite enzyme est une enzyme qui catalyse une réaction conduisant à un signal détectable.

7. Procédé selon la revendication 6, dans lequel ladite enzyme est une nitroréductase, une luciférase, une bêta galactosidase, l'aequorine, une bêta glucuronidase, une bêta lactamase ou une phosphatase alcaline.

8. Procédé selon la revendication 1, dans lequel (Z) est une protéine ou partie de protéine qui se lie de manière spécifique à un anticorps ou fragment d'anticorps de liaison localisé à l'intérieur de ladite cellule, ou une partie épitopique de ladite protéine.

9. Procédé selon la revendication 1, dans lequel (Z) est un anticorps ou fragment d'anticorps de liaison qui se lie de manière spécifique à une protéine épitopique localisée à l'intérieur de ladite cellule.

10. Cellule recombinante, transformée ou transfectée avec des molécules d'acide nucléique (a) et (b) telles que définies par l'une quelconque des revendications 1 et 3 à 9.

11. Cellule selon la revendication 10, dans laquelle une molécule d'acide nucléique ou les deux sont incorporées de manière stable dans le génome de la cellule.

12. Cellule selon l'une des revendications 10 ou 11, dans laquelle la première protéine d'essai est une protéine liée à la membrane, telle qu'un récepteur transmembranaire, un GPCR, un récepteur cellulaire, un canal ionique, un récepteur de facteur de croissance ou un récepteur de cytokine.

13. Coffret d'essai utile pour déterminer si un composé d'essai module une interaction spécifique protéine/protéine d'intérêt, comprenant une partie séparée de chacun des produits de construction de molécule d'acide nucléique (a) et (b) tels que définis par l'une quelconque des revendications 1 et 3 à 9, facultativement dans lequel la première protéine d'essai est une protéine liée à la membrane, telle qu'un récepteur transmembranaire et/ou la seconde protéine est une protéine inhibitrice, telle qu'une arrestine.
